# EUROPÄISCHE PATENTSCHRIFT

(11) **EP 2 760 825 B1**
(45) Veröffentlichungstag und Bekanntmachung des Hinweises auf die Patenterteilung: **14.09.2016**
(21) Anmeldenummer: 12762304.9
(22) Anmeldetag: 26.09.2012
(51) Int. Cl.: C07C 281/20, C07D 295/215, C12Q 1/26, C12Q 1/32

(54) **AZOMEDIATOREN**
AZO MEDIATORS
AZOMÉDIATEURS

(30) Priorität: 28.09.2011 EP 11183111
(43) Veröffentlichungstag der Anmeldung: 06.08.2014
(73) Patentinhaber: F.Hoffmann-La Roche AG, 4070 Basel (CH); Roche Diabetes Care GmbH, 68305 Mannheim (DE)
(72) Erfinder: DUVALL, Stacy H., Indianapolis, Indiana 46256 (US); GEBAUER, Peter, 82377 Penzberg (DE); HEINDL, Dieter, 82396 Paehl (DE); HORN, Carina, 68647 Biblis (DE); KRATZSCH, Peter, 82377 Penzberg (DE); MARQUANT, Michael, 68309 Mannheim (DE); MEIER, Thomas, 81373 München (DE)
(74) Vertreter: Weickmann & Weickmann PartmbB
(86) Internationale Anmeldenummer: PCT/EP2012/068951
(87) Internationale Veröffentlichungsnummer: WO 2013/045487

(56) Entgegenhaltungen:
- JP-A- 10 265 691
- US-A- 5 206 147
- US-A- 5 286 362
- EDWARD M. KOSOWER ET AL: "Glutathione. 6. Probable mechanism of action of diazene antibiotics", JOURNAL OF MEDICINAL CHEMISTRY, Bd. 15, Nr. 3, 1. März 1972 (1972-03-01), Seiten 307-312, XP055013458, ISSN: 0022-2623, DOI: 10.1021/jm00273a023

## Beschreibung

Die vorliegende Anmeldung betrifft Azoverbindungen sowie deren Verwendung als Mediator in einem diagnostischen Verfahren. Weiterhin betrifft die vorliegende Anmeldung Nachweisreagenzien, Kits und Testelemente, welche derartige Azoverbindungen umfassen.

Diagnostische Elemente stellen wichtige Bestandteile klinisch relevanter Verfahren zur Bestimmung von Analyten dar. Hierbei steht insbesondere eine schnelle und präzise Messung von Analyten im Vordergrund, welche unter Verwendung enzymbasierter Systeme bestimmt werden. Hierbei wird der zu bestimmende Analyt mit einem geeigneten, üblicherweise in katalytischen Mengen eingesetzten Enzym, einem Coenzym und gegebenenfalls einem Mediator in Kontakt gebracht, wobei das Coenzym physikochemisch verändert, z.B. oxidiert oder reduziert, wird und in der Folge mittels geeigneter Techniken detektiert werden kann.

Sofern zusätzlich ein Mediator zum Einsatz gelangt, überträgt dieser die bei der enzymatischen Umsetzung des Analyten freigesetzten Redoxäquivalente vom physikochemisch veränderten Coenzym im Allgemeinen auf einen optischen Indikator oder auf die leitfähigen Bestandteile einer Elektrode, so dass der Vorgang beispielsweise photometrisch oder elektrochemisch erfasst werden kann. Eine Kalibrierung des Messwerts liefert alsdann einen direkten Zusammenhang mit der Konzentration des zu bestimmenden Analyten.

Mediatoren, welche in Testelementen zur Bestimmung von Analyten zum Einsatz gelangen, sind im Stand der Technik bekannt und umfassen u.a. Kaliumhexacyanoferrat, Ferrocen-Derivate, Chinone, Oxazine, Phenazine und Thiazine. Eine Übersicht über Mediatoren, welche eine direkte Übertragung von Redoxäquivalenten auf ein geeignetes Detektionssystem gewährleisten und speziell bei der elektrochemischen Bestimmung von Blutglucose verwendet werden können, findet sich beispielsweise in Takaminami (Materials Integration (2008), 21, 317-323) und Heller et al. (Chemical Reviews (2008), 108, 2482-2505).

Eine andere bekannte Klasse von Mediatoren umfasst Nitrosoaniline, wie sie beispielsweise in US 5,206,147 und US 5,286,362 offenbart sind. Nitrosoaniline zeichnen sich gegenüber oben beschriebenen Mediatoren, welche eine direkte Übertragung von Redoxäquivalenten ermöglichen, dadurch aus, dass zunächst eine Vorläuferverbindung des eigentlichen Mediators bereitgestellt und diese erst in einem Folgeschritt zum effektiven Mediator reduziert wird, was den Vorteil einer Vermeidung von Blindreaktionen mit sich bringt.

Bei der Verwendung von Nitrosoanilinen stellt sich allerdings das Problem, dass diese allgemein zur Bildung von Azoxydimeren neigen und eine hohe Reaktivität gegenüber Aminogruppen-haltigen bzw. Mercaptogruppen-haltigen Substanzen aufweisen (J.A. Hinson; Advances in Experimental Medicine and Biology (1986), 197, 691-696), was letztlich zu unerwünschten Nebenreaktionen führen kann und die Spezifität und Sensitivität der Analytbestimmung negativ beeinflusst.

Die der Erfindung zugrunde liegende Aufgabe bestand somit darin, Mediatoren für die qualitative oder/und quantitative Bestimmung von Analyten bereitzustellen, bei welchen die Nachteile des Standes der Technik zumindest teilweise beseitigt sind. Insbesondere sollten die Mediatoren derart ausgestaltet sein, dass das Auftreten von Blindreaktionen, wie beispielsweise eine Reduktion des Mediators durch Ascorbinsäure, vermieden und gleichzeitig die Bildung von Nebenprodukten, wie beispielsweise von Dimeren des Mediators oder von Produkten einer unkontrollierten Umsetzung von Mediator und einer Aminogruppen-haltigen oder/und Mercaptogruppen-haltigen Substanz, minimiert wird. Ferner sollten die Mediatoren bei annehmbarer Reaktionsgeschwindigkeit eine gegenüber Nitrosoanilinen erhöhte Hydrolysestabilität aufweisen.

Diese Aufgabe wurde erfindungsgemäß gelöst mittels Azoverbindungen der allgemeinen Formel (I) 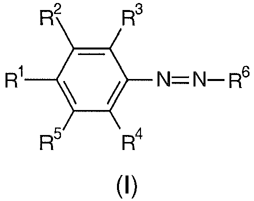 worin
R¹ einen Rest ausgewählt aus der Gruppe bestehend aus OR⁷ und NR⁷R⁸ bedeutet;
R², R³, R⁴ und R⁵ unabhängig voneinander einen Rest ausgewählt aus der Gruppe bestehend aus H, Alkyl, Cycloalkyl, Alkenyl, Alkinyl, Aryl, Heterocycloalkyl, Heteroaryl, CN, COO⁻, Halogen, O(Alkyl), O(Aryl), NH(Alkyl), N(Alkyl)₂, N(Alkyl)₃⁺, NH(Aryl), N(Aryl)₂, NO₂ und SO₃⁻ bedeuten, wobei das Alkyl, Cycloalkyl, Alkenyl, Alkinyl, Aryl, Heterocycloalkyl, Heteroaryl, O(Alkyl), O(Aryl), NH(Alkyl), N(Alkyl)₂, N(Alkyl)₃⁺, NH(Aryl) oder N(Aryl)₂ einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfassen kann;
R⁶ einen Rest ausgewählt aus der Gruppe bestehend aus C(=X)NR⁹R¹⁰, C(=X)NHOH und C(=X)R⁹ bedeutet;
R⁷ einen Rest ausgewählt aus der Gruppe bestehend aus Alkyl, Cycloalkyl, Alkenyl, Alkinyl, Aryl, Heterocycloalkyl und Heteroaryl bedeutet, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst,
R⁸ einen Rest ausgewählt aus der Gruppe bestehend aus Alkyl, Cycloalkyl, Alkenyl, Alkinyl, Aryl, Heterocycloalkyl und Heteroaryl bedeutet, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²- und SO₃⁻ umfassen kann, oder
R⁷ und R⁸ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S umfassen kann und einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst;
R⁹ und R¹⁰ unabhängig voneinander einen Rest ausgewählt aus der Gruppe bestehend aus Alkyl, Cycloalkyl, Alkenyl, Akinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O(Alkyl), O-(CH₂CH₂)ₙ-OH, O-(CH₂CH₂)ₙ-O(Alkyl), O(Aralkyl), O(Aryl), OPO₃²⁻, PO₃²⁻ und SO₃⁻ umfassen kann, oder
R⁹ und R¹⁰ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O(Alkyl), O-(CH₂CH₂)ₙ-OH, O-(CH₂CH₂)ₙ-O(Alkyl), O(Aralkyl), O(Aryl), OPO₃²⁻, PO₃²⁻ und SO₃⁻ umfassen kann;
X ein Heteroatom ausgewählt aus der Gruppe bestehend aus O und S bedeutet; und
n eine ganze Zahl von 1 bis 6 bedeutet;
mit der Maßgabe, dass mindestens zwei Reste ausgewählt aus der Gruppe bestehend aus R², R³, R⁴ und R⁵ den Rest H bedeuten.

Der Begriff "Alkyl", wie er im Rahmen der vorliegenden Anmeldung verwendet wird, bezeichnet einen gesättigten, linearen oder verzweigten Kohlenwasserstoffrest mit 1-12 Kohlenstoffatomen, welcher eine Bindungsvalenz an einem beliebigen der 1-12 Kohlenstoffatome aufweist. Bevorzugt stellt Alkyl einen Kohlenwasserstoffrest mit 1-8 Kohlenstoffatomen, stärker bevorzugt mit 1-6 Kohlenstoffatomen, dar. Besonders bevorzugte Alkyle umfassen Methyl, Ethyl, n-Propyl, iso-Propyl, n-Butyl, iso-Butyl, sec-Butyl und tert-Butyl.

Der Begriff "Cycloalkyl", wie er im Rahmen der vorliegenden Anmeldung verwendet wird, bezeichnet einen gesättigten oder ungesättigten, cyclischen Kohlenwasserstoffrest mit 3-12 Kohlenstoffatomen, welcher eine Bindungsvalenz an einem beliebigen der 3-12 Kohlenstoffatome aufweist. Bevorzugt stellt Cycloalkyl einen cyclischen Kohlenwasserstoffrest mit 3-10 Kohlenstoffatomen, stärker bevorzugt mit 3-8 Kohlenstoffatomen, dar. Besonders bevorzugte Cycloalkyle umfassen Cyclopropyl, Cyclobutyl, Cyclopentyl, Cyclohexyl, Cycloheptyl und Cyclooctyl.

Der Begriff "Alkenyl", wie er im Rahmen der vorliegenden Anmeldung verwendet wird, bezeichnet einen ungesättigten, linearen oder verzweigten Kohlenwasserstoffrest mit 2-12 Kohlenstoffatomen, welcher eine Bindungsvalenz an einem beliebigen der 2-12 Kohlenstoffatome und mindestens eine Doppelbindung aufweist. Bevorzugt stellt Alkenyl einen Kohlenwasserstoffrest mit 2-8 Kohlenstoffatomen, stärker bevorzugt mit 2-6 Kohlenstoffatomen, dar. Besonders bevorzugte Alkenyle umfassen Ethenyl, Propenyl und Butenyl.

Der Begriff "Alkinyl", wie er im Rahmen der vorliegenden Anmeldung verwendet wird, bezeichnet einen ungesättigten, linearen oder verzweigten Kohlenwasserstoffrest mit 2-12 Kohlenstoffatomen, welcher eine Bindungsvalenz an einem beliebigen der 2-12 Kohlenstoffatome und mindestens eine Dreifachbindung aufweist. Bevorzugt stellt Alkinyl einen Kohlenwasserstoffrest mit 2-8 Kohlenstoffatomen, stärker bevorzugt mit 2-6 Kohlenstoffatomen, dar. Besonders bevorzugte Alkinyle umfassen Ethinyl, Propinyl und Butinyl.

Der Begriff "Aryl", wie er im Rahmen der vorliegenden Anmeldung verwendet wird, bezeichnet ein aromatisches Ringsystem mit 3-14 Ringatomen, stärker bevorzugt mit 6-10 Ringatomen, welches als Ringatome ausschließlich Kohlenstoffatome enthält und eine Bindungsvalenz an einem beliebigen Kohlenstoffatom der 3-14 ringbildenden Atome aufweist. Bevorzugte Aryle umfassen Phenyl, Naphthyl, Anthracenyl und Phenanthrenyl.

Der Begriff "Aralkyl", wie er im Rahmen der vorliegenden Anmeldung verwendet wird, bezeichnet einen vorstehend definierten Alkylrest, in welchem mindestens ein Wasserstoffatom durch einen vorstehend definierten Arylrest ersetzt ist. Bevorzugte Aralkyle umfassen Benzyl und Phenethyl.

Der Begriff "Heterocycloalkyl", wie er im Rahmen der vorliegenden Anmeldung verwendet wird, bezeichnet ein gesättigtes oder teilweise ungesättigtes Ringsystem mit 3-14 Ringatomen, stärker bevorzugt mit 5-7 Ringatomen, welches als Ringatome neben Kohlenstoffatomen mindestens ein Heteroatom ausgewählt aus der Gruppe bestehend aus N, O und S enthält und eine Bindungsvalenz an einem beliebigen Kohlenstoffatom oder Stickstoffatom der 3-14 ringbildenden Atome aufweist. Bevorzugte Heterocycloalkyle umfassen Azepinyl, Dihydrofuryl, Dihydropyranyl, Imidazolidinyl, Imidazolinyl, Isothiazolidinyl, Isoxazolidinyl, Morpholinyl, Oxazolidinyl, Piperazinyl, Piperidinyl, Pyrazolidinyl, Pyrrolidinyl, Tetrahydrofuryl, Tetrahydropyranyl, Thiadiazolylidinyl, Thiazolidinyl und Thiomorpholinyl.

Der Begriff "Heteroaryl", wie er im Rahmen der vorliegenden Anmeldung verwendet wird, bezeichnet ein aromatisches Ringsystem mit 3-14 Ringatomen, stärker bevorzugt mit 5-6 Ringatomen, welches als Ringatome neben Kohlenstoffatomen mindestens ein Heteroatom ausgewählt aus der Gruppe bestehend aus N, O und S enthält und eine Bindungsvalenz an einem beliebigen Kohlenstoffatom oder Stickstoffatom der 3-14 ringbildenden Atome aufweist. Bevorzugte Heteroaryle umfassen Furanyl, Imidazolyl, Isothiazolyl, Isoxazolyl, Oxadiazolyl, Oxazolyl, Pyrazolyl, Pyrrolyl, Tetrazolyl, Thiadiazolyl, Thiazolyl, Thienyl, Triazolyl, Pyrazinyl, Pyridazinyl, Pyridyl, Pyrimidinyl und Triazinyl.

Der Begriff "Halogen", wie er im Rahmen der vorliegenden Anmeldung verwendet wird, umfasst Fluor, Chlor, Brom und Iod.

In den erfindungsgemäßen Azoverbindungen der allgemeinen Formel (I) bedeutet R¹ in einer bevorzugten Ausführungsform den Rest NR⁷R⁸.

In einer anderen bevorzugten Ausführungsform der Erfindung bedeuten mindestens drei Reste ausgewählt aus der Gruppe bestehend aus R², R³, R⁴ und R⁵ den Rest H, während der vierte Rest einen beliebigen Rest ausgewählt aus der Gruppe bestehend aus H, Alkyl, Cycloalkyl, Alkenyl, Alkinyl, Aryl, Heterocycloalkyl, Heteroaryl, CN, COO⁻, Halogen, O(Alkyl), O(Aryl), NH(Alkyl), N(Alkyl)₂, N(Alkyl)₃⁺, NH(Aryl), N(Aryl)₂, NO₂ und SO₃⁻ bedeuten kann, wobei das Alkyl, Cycloalkyl, Alkenyl, Alkinyl, Aryl, Heterocycloalkyl, Heteroaryl, O(Alkyl), O(Aryl), NH(Alkyl), N(Alkyl)₂, N(Alkyl)₃⁺, NH(Aryl) oder N(Aryl)₂ wie oben definiert substituiert sein kann. Sofern in den erfindungsgemäßen Azoverbindungen der allgemeinen Formel (I) mindestens drei Reste ausgewählt aus der Gruppe bestehend aus R², R³, R⁴ und R⁵ den Rest H bedeuten, so ist es als besonders bevorzugt anzusehen, dass R² einen Rest ausgewählt aus der Gruppe bestehend aus H und O(Alkyl) bedeutet, und R³, R⁴ und R⁵ jeweils den Rest H bedeuten. Am stärksten bevorzugt bedeuten die Reste R², R³, R⁴ und R⁵ allesamt jeweils H.

In einer weiteren bevorzugten Ausführungsform der Erfindung bedeutet R⁶ einen Rest ausgewählt aus der Gruppe bestehend aus C(=X)NR⁹R¹⁰ und C(=X)NHOH, wobei C(=X)NR⁹R¹⁰ als bevorzugt anzusehen ist. Am stärksten bevorzugt handelt es sich bei R⁶ um den Rest C(=O)NR⁹R¹⁰.

In einer weiteren bevorzugten Ausführungsform der Erfindung bedeutet R⁷ einen Alkylrest, wobei das Alkyl einen oder mehrere hydrophile Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst, wodurch die Wasserlöslichkeit der Azoverbindungen verbessert werden kann. Stärker bevorzugt umfasst das Alkyl als hydrophile(n) Substituenten eine oder mehrere OH-Gruppen, und insbesondere eine einzige OH-Gruppe.

In einer weiteren bevorzugten Ausführungsform der Erfindung bedeutet R⁸ einen Rest ausgewählt aus der Gruppe bestehend aus Alkyl, Cycloalkyl, Alkenyl, Alkinyl, Aryl, Heterocycloalkyl und Heteroaryl, wobei das Alkyl, Cycloalkyl, Alkenyl, Alkinyl, Aryl, Heterocycloalkyl oder Heteroaryl einen oder mehrere hydrophile Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst.

Stärker bevorzugt bedeutet R⁸ einen Alkylrest, wobei das Alkyl einen oder mehrere hydrophile Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst. Stärker bevorzugt umfasst das Alkyl als hydrophile(n) Substituenten eine oder mehrere OH-Gruppen, und insbesondere eine einzige OH-Gruppe.

In der am stärksten bevorzugten Ausführungsform bedeutet R⁷ oder/und R⁸, insbesondere R⁷ und R⁸, den Rest Hydroxyethyl.

In einer weiteren bevorzugten Ausführungsform der Erfindung bedeuten R⁹ und R¹⁰ unabhängig voneinander einen Rest ausgewählt aus der Gruppe bestehend aus Alkyl und Aryl, wobei das Alkyl oder Aryl einen oder mehrere Substituenten ausgewählt aus der Gruppe bestehend aus OH, O(Alkyl) und O(Aryl) umfassen kann. Stärker bevorzugt bedeuten R⁹ und R¹⁰ unabhängig voneinander einen Rest ausgewählt aus der Gruppe bestehend aus Methyl, Ethyl, Phenyl, Hydroxymethyl, Hydroxyethyl, Hydroxyphenyl und Methoxyphenyl, wobei Methyl, Ethyl und Hydroxyethyl als bevorzugt anzusehen sind.

In einer alternativen Ausführungsform der Erfindung bilden R⁹ und R¹⁰ zusammen mit dem N-Atom, an welches sie gebunden sind, einen 5- oder 6-gliedrigen Heterocyclus, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O(Alkyl), O-(CH₂CH₂)ₙ-OH, O-(CH₂CH₂)ₙ-O(Alkyl), O(Aralkyl), O(Aryl), OPO₃²⁻, PO₃²⁻ und SO₃⁻ umfassen kann.

Sofern der 5- oder 6-gliedrigen Heterocyclus substituiert ist, so ist es bevorzugt, dass der Heterocyclus einen oder mehrere hydrophile Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst.

In noch einer weiteren bevorzugten Ausführungsform der Erfindung bedeutet X ein Sauerstoffatom.

Die erfindungsgemäßen Azoverbindungen der allgemeinen Formel (I), bei welchem es sich vorzugsweise nicht um 2-(Ethyl-{3-methyl-4-[(piperidin-1-ylcarbonyl)diazenyl]phenyl}amino)ethanol handelt, können naturgemäß, im Gegensatz zu Nitrosoanilin-Mediatoren, keine Azoxydimere bilden. Ferner verfügen die erfindungsgemäßen Azoverbindungen über keinerlei reaktive Nitrosogruppen, über welche eine Reaktion mit Aminogruppen-haltigen oder/und Mercaptogruppen-haltigen Substanzen, wie beispielsweise mit Enzymen, erfolgen könnte.

Andererseits stellen Azoverbindungen der allgemeinen Formel (I), in Analogie zu Nitrosoanilinen, ausschließlich Vorläuferverbindungen dar, aus welchen erst durch nachfolgende Reduktion der eigentliche Mediator gebildet wird. Somit weisen die erfindungsgemäßen Azoverbindungen zum einen den für Nitrososaniline bekannten Vorteil der Vermeidung von Blindreaktionen auf, während gleichzeitig unerwünschte Nebenreaktionen, wie sie bei einer Analytbestimmung mittels Nitrosoanilinen beobachtet werden, vermieden werden können.

Insofern betrifft die vorliegende Erfindung in einem weiteren Aspekt die Verwendung einer Azoverbindung der allgemeinen Formel (I) 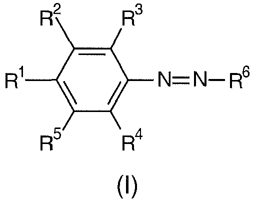 als Mediator in einem diagnostischen Verfahren, worin
R¹ einen Rest ausgewählt aus der Gruppe bestehend aus OH, OR⁷, SR⁷, NHR⁷ und NR⁷R⁸ bedeutet;
R², R³, R⁴ und R⁵ unabhängig voneinander einen Rest ausgewählt aus der Gruppe bestehend aus H, Alkyl, Cycloalkyl, Alkenyl, Alkinyl, Aryl, Heterocycloalkyl, Heteroaryl, CN, COO⁻, Halogen, O(Alkyl), O(Aryl), NH(Alkyl), N(Alkyl)₂, N(Alkyl)₃⁺, NH(Aryl), N(Aryl)₂, NO₂ und SO₃⁻ bedeuten, wobei das Alkyl, Cycloalkyl, Alkenyl, Alkinyl, Aryl, Heterocycloalkyl, Heteroaryl, O(Alkyl), O(Aryl), NH(Alkyl), N(Alkyl)₂, N(Alkyl)₃⁺, NH(Aryl) oder N(Aryl)₂ einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, 0-(CH₂CH₂)ₙ-O(Alkyl), O(Alkyl), O(Aralkyl), O(Aryl), OPO₃²⁻, PO₃²⁻ und SO₃⁻ umfassen kann;
R⁶ einen Rest ausgewählt aus der Gruppe bestehend aus CN, C(=X)NH₂, C(=_{X})NHR⁹, C(₌X)NR⁹R¹⁰, C(=X)NHOH und C(=X)R⁹ bedeutet;
R⁷ und R⁸ unabhängig voneinander einen Rest ausgewählt aus der Gruppe bestehend aus Alkyl, Cycloalkyl, Alkenyl, Alkinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann, oder
R⁷ und R⁸ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann;
R⁹ und R¹⁰ unabhängig voneinander einen Rest ausgewählt aus der Gruppe bestehend aus Alkyl, Cycloalkyl, Alkenyl, Akinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann, oder
R⁹ und R¹⁰ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, 0 und S oder/und einen oder mehrere Substituenten ausgewählt aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann;
X ein Heteroatom ausgewählt aus der Gruppe bestehend aus O und S bedeutet; und
n eine ganze Zahl von 1 bis 6 bedeutet.

Was bevorzugte Ausführungsformen der als Mediator eingesetzten Azoverbindungen betrifft, so wird auf die Ausführungen in Zusammenhang mit der Beschreibung der erfindungsgemäßen Azoverbindungen verwiesen.

In ihrer Funktion als Mediator können oben beschriebene Azoverbindungen grundsätzlich in jedem diagnostischen Verfahren zur Anwendung gelangen, welches eine qualitative oder/und quantitative Bestimmung eines Analyten in einer Probe ermöglicht, wie beispielsweise in einem optischen oder elektrochemischen diagnostischen Verfahren. Hierbei können die Azoverbindungen als Monomere oder Polymere vorliegen und sowohl in freier als auch in gebundener Form zum Einsatz gelangen. So ist es insbesondere möglich, die Azoverbindungen auf einem Träger, wie beispielsweise auf einer Elektrode, zu immobilisieren oder/und an andere Bestandteile eines geeigneten Nachweisreagenzes, wie beispielsweise an ein Enzym oder Coenzym, zu koppeln.

Vorzugsweise werden die oben beschriebenen Azoverbindungen in Form eines Nachweisreagenzes eingesetzt, welches neben der als Mediator fungierenden Azoverbindung weitere Komponenten, wie beispielsweise Enzyme, Coenzyme und optische Indikatoren, umfassen kann.

Dementsprechend betrifft die Erfindung in noch einem weiteren Aspekt ein Nachweisreagenz zur Bestimmung eines Analyten, umfassend
(a) eine Flavin-, Nicotinamid- oder Pyrrolochinolinchinon-abhängige Oxidoreduktase,
(b) ein reduzierbares Flavin-, Nicotinamid- oder Pyrrolochinolinchinon-Coenzym,
(c) eine Azoverbindung der allgemeinen Formel (I) 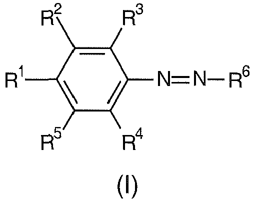 worin
   R¹ einen Rest ausgewählt aus der Gruppe bestehend aus OH, OR⁷, SR⁷, NHR⁷ und NR⁷R⁸ bedeutet;
   R², R³, R⁴ und R⁵ unabhängig voneinander einen Rest ausgewählt aus der Gruppe bestehend aus H, Alkyl, Cycloalkyl, Alkenyl, Alkinyl, Aryl, Heterocycloalkyl, Heteroaryl, CN, COO⁻, Halogen, O(Alkyl), O(Aryl), NH(Alkyl), N(Alkyl)₂, N(Alkyl)₃⁺, NH(Aryl), N(Aryl)₂, NO₂ und SO₃⁻ bedeuten, wobei das Alkyl, Cycloalkyl, Alkenyl, Alkinyl, Aryl, Heterocycloalkyl, Heteroaryl, O(Alkyl), O(Aryl), NH(Alkyl), N(Alkyl)₂, N(Alkyl)₃⁺, NH(Aryl) oder N(Aryl)₂ einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃ umfassen kann;
   R⁶ einen Rest ausgewählt aus der Gruppe bestehend aus CN, C(=X)NH₂, C(=X)NHR⁹, C(=X)NR⁹R¹⁰, C(=X)NHOH und C(=X)R⁹ bedeutet;
   R⁷ und R⁸ unabhängig voneinander einen Rest ausgewählt aus der Gruppe bestehend aus Alkyl, Cycloalkyl, Alkenyl, Alkinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann, oder
   R⁷ und R⁸ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann;
   R⁹ und R¹⁰ unabhängig voneinander einen Rest ausgewählt aus der Gruppe bestehend aus Alkyl, Cycloalkyl, Alkenyl, Akinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann, oder
   R⁹ und R¹⁰ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, 0-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann;
   X ein Heteroatom ausgewählt aus der Gruppe bestehend aus O und S bedeutet; und
   n eine ganze Zahl von 1 bis 6 bedeutet, und
(d) gegebenenfalls einen reduzierbaren optischen Indikator.

Was bevorzugte Ausführungsformen der in dem erfindungsgemäßen Nachweisreagenz enthaltenen Azoverbindungen betrifft, so wird auf die Ausführungen in Zusammenhang mit der Beschreibung der erfindungsgemäßen Azoverbindungen verwiesen.

Das in dem erfindungsgemäßen Nachweisreagenz eingesetzte Enzym kann grundsätzlich jede beliebige Flavin-, Nicotinamid- oder Pyrrolochinolinchinon-abhängige Oxidoreduktase sein, welche dem Fachmann für die Zwecke der jeweiligen Anwendung geeignet erscheint. Vorzugsweise handelt es sich bei der Flavin-, Nicotinamid- oder Pyrrolochinolinchinon-abhängigen Oxidoreduktase um eine Flavin-, Nicotinamid- oder Pyrrolochinolinchinon-abhängige Dehydrogenase, wie beispielsweise um eine Alkohol-Dehydrogenase, Glucose-Dehydrogenase, Glucose-6-phosphat-Dehydrogenase, Glycerin-Dehydrogenase, Lactat-Dehydrogenase oder Malat-Dehydrogenase.

In einer stärker bevorzugten Ausführungsform der Erfindung wird als Flavin-, Nicotinamid- oder Pyrrolochinolinchinon-abhängigen Oxidoreduktase eine Flavin-, Nicotinamid- oder Pyrrolochinolinchinon-abhängige Glucose-Dehydrogenase oder Glucose-6-phosphat-Dehydrogenase eingesetzt. Am stärksten bevorzugt umfasst das Nachweisreagenz eine Nicotinamidabhängige Glucose-Dehydrogenase oder Glucose-6-phosphat-Dehydrogenase.

Neben dem Enzym umfasst das erfindungsgemäße Nachweisreagenz weiterhin ein reduzierbares Flavin-, Nicotinamid- oder Pyrrolochinolinchinon-Coenzym, welches vom Fachmann unter Berücksichtigung des jeweils eingesetzten Enzyms in geeigneter Weise ausgewählt werden kann. Vorzugsweise handelt es sich bei dem reduzierbaren Flavin-, Nicotinamid- oder Pyrrolochinolinchinon-Coenzym um FAD, FMN, NAD⁺, NADP⁺, PQQ, oder um Derivate hiervon, wobei NAD⁺, NADP⁺ und Derivate hiervon als bevorzugt anzusehen sind.

Sofern Derivate von NAD⁺ oder NADP⁺ zum Einsatz gelangen, so handelt es sich bevorzugt um stabilisierte Nikotinamid-Adenin-Dinukleotid(phosphat)-Verbindungen, d.h. um chemische Derivate von nativem Nikotinamid-Adenin-Dinukleotid(phosphat). Stabilisierte Nikotinamid-Adenin-Dinukleotid-(phosphat)-Verbindungen im Sinne der vorliegenden Erfindung, welche insbesondere carbacyclische Analoga wie carbaNAD⁺ und carbaNADP⁺ umfassen, sind u.a. in Slama (Biochemistry (1988), 27, 183-193), Blackburn et al. (Chemical Communications (1996), 2765-2766), US 5,801,006, WO 98/33936, WO 01/49247 und WO 2007/012494 beschrieben. In der am stärksten bevorzugten Ausführungsform handelt es sich bei dem reduzierbaren Flavin-, Nicotinamid- oder Pyrrolochinolinchinon-Coenzym um NAD⁺, NADP⁺, carbaNAD⁺ oder carbaNADP⁺.

Sofern gewünscht, kann das erfindungsgemäße Nachweisreagenz neben dem Enzym, dem Coenzym und der Azoverbindung zusätzlich einen reduzierbaren optischen Indikator umfassen, welcher vom Fachmann entsprechend den Anforderungen an das Nachweisreagenz ausgewählt werden kann. Als optischer Indikator kann eine beliebige Substanz zum Einsatz gelangen, welche reduzierbar ist und bei Reduktion eine visuell oder/und maschinell detektierbare Änderung ihrer optischen Eigenschaften, wie beispielsweise Farbe, Fluoreszenz, Remission, Transmission, Polarisation oder/und Brechungsindex, erfährt. Bevorzugte optische Indikatoren im Sinne der vorliegenden Erfindung umfassen reduzierbare Heteropolysäuren, insbesondere 2,18-Phosphormolybdänsäure.

Das erfindungsgemäße Nachweisreagenz kann zur qualitativen oder/und quantitativen Bestimmung einer beliebigen biologischen oder chemischen Substanz verwendet werden, welche optisch oder elektrochemisch nachweisbar ist. Vorzugsweise wird der Analyt aus der Gruppe bestehend aus Äpfelsäure, Alkohol, Ascorbinsäure, Cholesterin, Glucose, Glycerin, Harnstoff, 3-Hydroxybutyrat, Milchsäure, Pyruvat und Triglyceriden ausgewählt, wobei Glucose besonders bevorzugt ist.

Der zu bestimmende Analyt kann aus einer beliebigen Quelle stammen, ist jedoch bevorzugt in einem Körperfluid, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie beispielsweise Speichel oder Schweiß, enthalten. Bevorzugt wird mittels des erfindungsgemäßen Nachweisreagenzes indessen das Vorhandensein oder/und die Menge eines Analyten in einer Probe ausgewählt aus der Gruppe bestehend aus Vollblut, Plasma und Serum bestimmt.

In noch einem weiteren Aspekt betrifft die Erfindung einen Kit zur Bestimmung eines Analyten, welcher ein erfindungsgemäßes Nachweisreagenz und ein Testelement umfasst. In einer bevorzugten Ausführungsform der Erfindung ist das Testelement als optischer oder elektrochemischer Sensor ausgebildet, welcher beispielsweise einen Applikationsbereich zum Aufbringen der Probe, einen Reaktionsbereich zum Umsetzen des Analyten mit dem Nachweisreagenz, einen Detektionsbereich zum Bestimmen des Vorhandenseins oder/und der Menge des Analyten in der Probe, und gegebenenfalls einen Abfallbereich umfassen kann.

Testelemente, welche im Rahmen der vorliegenden Erfindung Anwendung finden können, umfassen insbesondere Testelemente, auf die eine den Analyten enthaltende Probe in Form einer wässrigen oder nichtwässrigen Lösung aufgebracht werden kann. Bevorzugte Testelemente im Sinne der vorliegenden Anmeldung umfassen Testbänder, Testdiscs, Testpads und Teststreifen, wobei Teststreifen als besonders bevorzugt anzusehen sind. Konkrete Beispiele für erfindungsgemäß eingesetzte Testelemente sind u.a. in US 5,271,895, US 6,207,000, US 6,540,890, US 6,755,949, US 7,008,799, US 7,025,836, US 7,067,320, US 2003/0031592 A1 und US 2006/0003397 A1 beschrieben.

In noch einem weiteren Aspekt betrifft die Erfindung ein Testelement zur Bestimmung eines Analyten, welches ein erfindungsgemäßes Nachweisreagenz umfasst. In einer bevorzugten Ausführungsform ist das Testelement als optischer oder elektrochemischer Sensor ausgebildet, welcher vorzugsweise einen Applikationsbereich zum Aufbringen der Probe, einen das erfindungsgemäße Nachweisreagenz enthaltenden Reaktionsbereich zum Umsetzen des Analyten mit dem Nachweisreagenz, einen Detektionsbereich zum Bestimmen des Vorhandenseins oder/und der Menge des Analyten in der Probe, und gegebenenfalls einen Abfallbereich umfasst. Was weitere bevorzugte Ausführungsformen der erfindungsgemäßen Testelemente betrifft, so wird auf die Ausführungen in Zusammenhang mit der Beschreibung der erfindungsgemäßen Kits verwiesen.

In noch einem weiteren Aspekt betrifft die Erfindung ein Verfahren zur Bestimmung eines Analyten, umfassend die Schritte:
(a) Inkontaktbringen einer den Analyten enthaltenden Probe mit einem erfindungsgemäßen Nachweisreagenz, einem erfindungsgemäßen Kit oder einem erfindungsgemäßen Testelement, und
(b) Bestimmen des Vorhandenseins oder/und der Menge des Analyten.

Im Rahmen des erfindungsgemäßen Verfahrens wird die den Analyten enthaltende Probe zunächst mit einem erfindungsgemäßen Nachweisreagenz, Kit oder Testelement in Kontakt gebracht, wobei der Analyt durch die Flavin-, Nicotinamid- oder Pyrrolochinolinchinon-abhängige Oxidoreduktase oxidiert wird, das reduzierbare Flavin-, Nicotinamid- oder Pyrrolochinolinchinon-Coenzym reduziert wird, und Elektronen des reduzierten Flavin-, Nicotinamid- oder Pyrrolochinolinchinon-Coenzyms durch die als Mediator eingesetzte Azoverbindung auf die leitfähigen Bestandteile einer Elektrode oder auf den gegebenenfalls vorhandenen optischen Indikator übertragen werden.

Zur qualitativen oder/und quantitativen Bestimmung des Analyten können alle aus dem Stand der Technik bekannten Methoden zum Nachweis enzymatischer Reaktionen eingesetzt werden, welche ein messbares Signal erzeugen, das vom Fachmann manuell oder/und mittels einer geeigneten Detektionsvorrichtung ausgewertet bzw. ausgelesen werden kann. Im Rahmen der vorliegenden Erfindung erfolgt die Bestimmung des Analyten bevorzugt optisch oder elektrochemisch, wobei optische Techniken insbesondere Photometrie und Fluorimetrie umfassen.

Die Erfindung soll durch die nachfolgenden Figuren und Beispiele näher erläutert werden.

### Beschreibung der Figuren

- **Figur 1:**: Cyclovoltagramm des Mediators 4-[Bis-(2-hydroxyethyl)-amino]-benzoldiazocarbamid (7) in Gegenwart von FAD-abhängiger Glucose-Dehydrogenase bei Glucosekonzentrationen von 0 mg/dl, 100 mg/dl, 200 mg/dl, 300 mg/dl, 400 mg/dl und 500 mg/dl. Spannungsänderung pro Zeiteinheit: 100 mV/sec
- **Figur 2:**: Kinetik der Umsetzung von Glucose unter Verwendung des Mediators 4-[Bis-(2-hydroxyethyl)-amino]-benzoldiazocarbamid (7) in Gegenwart von NAD-abhängiger Glucose-Dehydrogenase und carbaNAD bei Glucosekonzentrationen von 0 mg/dl, 100 mg/dl, 200 mg/dl, 300 mg/dl, 400 mg/dl und 500 mg/dl. Die Messung des Stromflusses nach einem Zeitraum von 5 sec, 10 sec, 15 sec bzw. 20 sec erfolgte jeweils bei einem angelegten Potential von 200 mV.
- **Figur 3:**: Kinetik der Reduktion von 4-[Bis-(2-hydroxyethyl)-amino]-benzoldiazocarbamid (7) mit verschiedenen Reduktionsmitteln. **3A:** Reduktion mit Ascorbinsäure (Messkurve ist zur besseren Erkennbarkeit mit einem Pfeil markiert) **3B:** Reduktion mit carbaNADH **3C:** Reduktion mit NADH

### Beispiele

### Beispiel 1: Synthese erfindungsgemäßer Azoverbindungen

Die eingesetzten Chemikalien sind, sofern nicht anders vermerkt, kommerziell erhältlich und von größtmöglicher handelsüblicher Qualität.

### Synthese von 2-[(2-Hydroxyethyl)-(4-nitrophenyl)-amino]-ethanol (3)

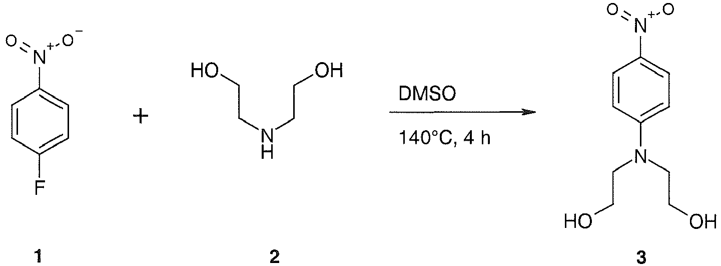

1-Fluornitrobenzol **(1)** (1.00 ml, 9.38 mmol) und Diethanolamin **(2)** (2.73 ml, 28.5 mmol) wurden in 20.0 ml DMSO gelöst und 4 Stunden bei 140 °C refluxiert. Im Anschluss wurde das Lösungsmittel unter vermindertem Druck (3 mbar, 80 °C) entfernt, wobei ein orangefarbenes Öl zurückblieb, welches in einem Gemisch aus Ethylacetat und Wasser gelöst wurde. Die organische Phase wurde abgetrennt, mehrfach mit Wasser gewaschen und über MgSO₄ getrocknet. Nach Entfernen des Lösungsmittels unter vermindertem Druck wurden 1.35 g (63%) der Titelverbindung erhalten.

### Synthese von N,N-Bis(2-hydroxyethyl)-4-aminoanilin Dihydrochlorid (4)

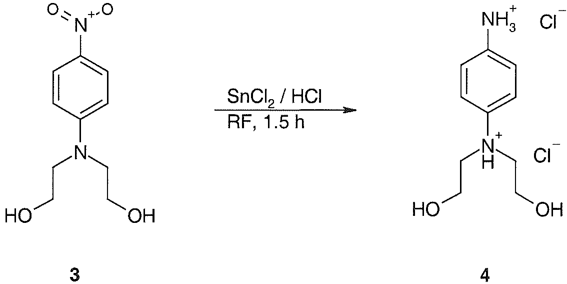

2-[(2-Hydroxyethyl)-(4-nitrophenyl)-amino]-ethanol **(3)** (1.35 g, 5.97 mmol) wurde in 100 ml konz. HCl (37%) gelöst, mit Zinn (II)-chlorid Dihydrat (8.07 g, 26.5 mmol) versetzt und für 1.5 h bei 105 °C refluxiert. Im Anschluss wurde das Gemisch durch Zugabe von Ammoniaklösung (25% in H₂O) unter Eiskühlung auf pH 8.5 eingestellt, wobei ein farbloser Feststoff ausfiel. Nach mehrfacher Extraktion der wässrigen Suspension mit Ethylacetat wurden die vereinigten organischen Phasen über Na₂SO₄ getrocknet, auf ein Viertel des ursprünglichen Volumens eingeengt und mit 3.00 ml konz. HCl (37%) versetzt. Nach Entfernen des Lösungsmittels unter vermindertem Druck und Trocknen des Rückstandes im Hochvakuum wurden 590.0 mg (37%) der Titelverbindung erhalten.

### Synthese von 4-[Bis-(2-hydroxyethyl)-amino]-benzoldiazocyanid (6)

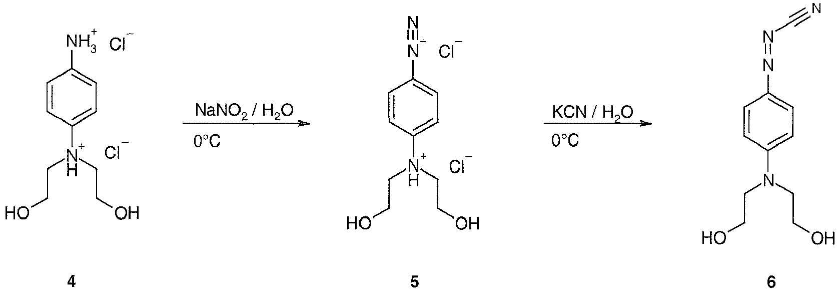

*N,N*-Bis(2-hydroxyethyl)-4-aminoanilin Dihydrochlorid **(4)** (50.0 mg, 0.186 mmol) wurde in 1.80 ml Wasser gelöst und auf 0 °C gekühlt. Im Anschluss wurde diese Lösung über einen Zeitraum von 30 min tropfenweise mit einer Lösung aus Natriumnitrit (38.5 mg, 0.558 mmol) in H₂O (1.90 ml) versetzt, woraufhin sich das Gemisch über rot nach grün-braun verfärbte. Anschließend wurde das Reaktionsgemisch unter Kühlung über einen Zeitraum von 30 min tropfenweise mit einer Lösung aus KCN (36.3 mg, 0.558 mmol) in H₂O (0.60 ml) versetzt, wobei eine Farbänderung nach rotbraun erfolgte. Nach 45 min Rühren bei dieser Temperatur wurde das Reaktionsgemisch mehrfach mit Ethylacetat extrahiert. Die vereinigten organischen Phasen wurden über Na₂SO₄ getrocknet und das Lösungsmittel unter vermindertem Druck entfernt. Eine chromatographische Reinigung des Rückstandes an Kieselgel (Eluent: CHCl₃/MeOH = 92:8) lieferte 31.4 mg (72%) der Titelverbindung als dunkelrote Kristalle.

### Synthese von 4-[Bis-(2-hydroxyethyl)-amino]-benzoldiazocarbamid (7)

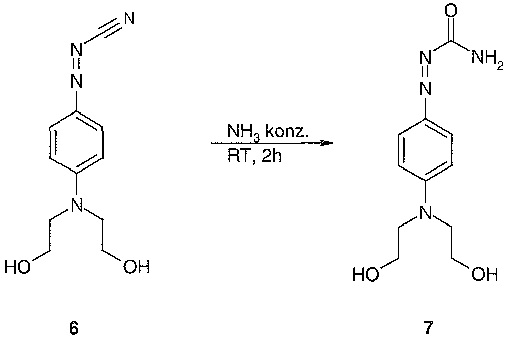

4-[Bis-(2-hydroxyethyl)-amino]-benzoldiazocyanid **(6)** (43.9 mg, 0.201 mmol) wurde in 15.0 ml Ammoniaklösung (30-33% in H₂O) gelöst und für 3 h bei Raumtemperatur gerührt. Nach Entfernen des Lösungsmittels unter vermindertem Druck wurde der Rückstand mittels präparativer HPLC (Hypersil ODS; 0.1 M Triethylammoniumacetat-Puffer/Acetonitril-Gradient) gereinigt, wobei 24.4 mg (38%) der Titelverbindung in Form des entsprechenden Acetatsalzes als dunkelrote, hygroskopische Kristalle erhalten wurden.

### Synthese von 4-[Bis-(2-hydroxyethyl)-amino]-benzoldiazodimethyl-carbamid (8)

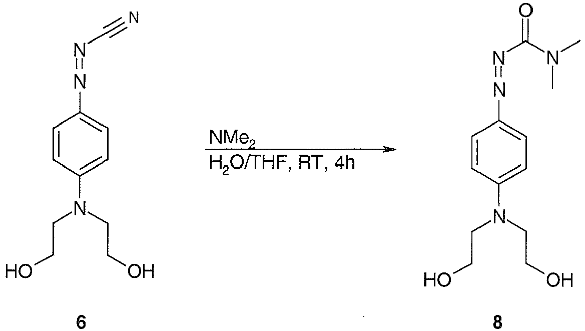

4-[Bis-(2-hydroxyethyl)-amino]-benzoldiazocyanid **(6)** (10.2 mg, 0.044 mmol) wurde in 2.00 ml H₂O gelöst. Im Anschluss wurde die Lösung mit Dimethylamin (2M in THF, 2.00 ml, 52.2 mmol) versetzt und das Reaktionsgemisch für 4 h bei Raumtemperatur gerührt. Nach Entfernen des Lösungsmittels unter vermindertem Druck wurde der Rückstand mittels präparativer HPLC (Hypersil ODS; 0.1 M Triethylammoniumacetat-Puffer/Acetonitril-Gradient) gereinigt, wobei 7.1 mg (47%) der Titelverbindung in Form des entsprechenden Monoacetatsalzes als dunkelrote, hygroskopische Kristalle erhalten wurden.

### Synthese von 4-[Bis-(2-hydroxyethyl)-amino]-benzoldiazo-bis-(2-hydroxyethyl)-carbamid (9)

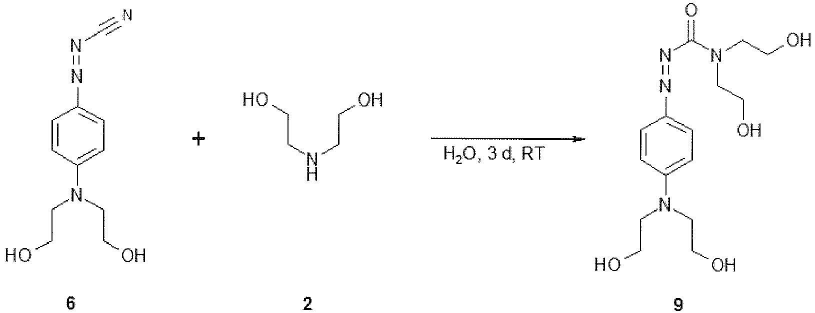

4-[Bis-(2-hydroxyethyl)-amino]-benzoldiazocyanid **(6)** (6.50 g, 27.75 mmol) wurde in 520 ml H₂O suspendiert. Im Anschluss wurde die Suspension mit Diethanolamin **(2)** (13.26 ml, 138.7 mmol) versetzt und das Reaktionsgemisch für 72 h bei Raumtemperatur gerührt. Nach Entfernen des Lösungsmittels unter vermindertem Druck wurde der Rückstand zweimal säulenchromatographisch an Kieselgel gereinigt (Eluent: CH₂Cl₂/MeOH = 85:15 + 2% NEt₃), gefolgt von einer weiteren säulenchromatographischen Reinigung an Sephadex LH20 (Eluent: H₂O). Nach Lyophilisation wurden 4.10 g (43%) der Titelverbindung als roter, amorpher Feststoff erhalten.

### Synthese von 4-(2-(4-(Bis-(2-hydroxyethyl-amino)-phenyl)-diazocarbonyl)-morpholin (11)

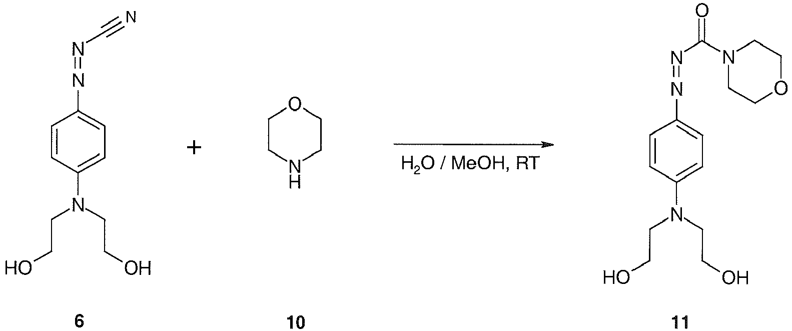

4-[Bis-(2-hydroxyethyl)-amino]-benzoldiazocyanid **(6)** (100.0 mg, 0.427 mmol) wurde in 4.00 ml H₂O/MeOH (1:1) suspendiert. Im Anschluss wurde die Suspension mit Morpholin (744 µl, 8.54 mmol) versetzt und das Reaktionsgemisch für 16 h bei Raumtemperatur gerührt. Nach Entfernen des Lösungsmittels unter vermindertem Druck wurde der Rückstand zweimal säulenchromatographisch an Kieselgel gereinigt (Eluent: CH₂Cl₂/MeOH = 70:30 + 2% NEt₃), wobei 12.5 mg (9%) der Titelverbindung als dunkelrote Kristalle erhalten wurden.

### Synthese von 2-(4-(Bis-(2-hydroxyethyl)-amino)-phenyl)-N-hydroxy-diazen-carboxamid (13)

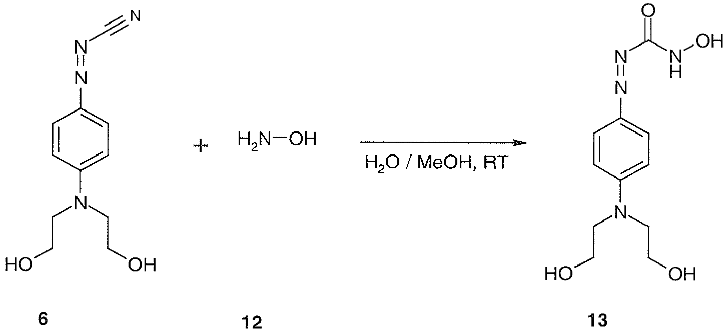

4-[Bis-(2-hydroxyethyl)-amino]-benzoldiazocyanid **(6)** (100.0 mg, 0.427 mmol) wurde in 4.00 ml H₂O/MeOH (1:1) suspendiert. Im Anschluss wurde die Suspension mit Hydroxylamin **(12)** (131 µl, 2.14 mmol) versetzt und das Reaktionsgemisch für 3 h bei Raumtemperatur gerührt. Nach Entfernen des Lösungsmittels unter vermindertem Druck wurde der Rückstand säulenchromatographisch an Kieselgel gereinigt (Eluent: CH₂Cl₂/MeOH = 85:15 + 2% NEt₃), wobei 53.1 mg (46%) der Titelverbindung als oranger Feststoff erhalten wurden.

### Synthese von 4-Methoxy-benzoldiazocyanid (16)

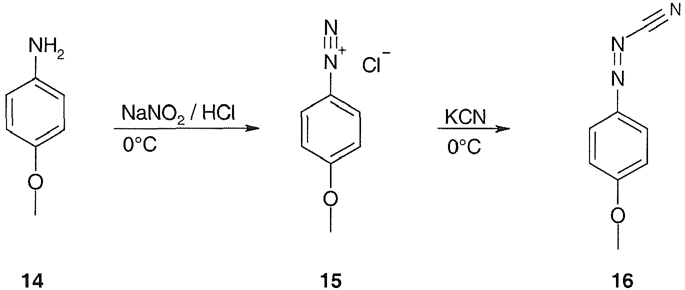

p-Anisidin **(14)** (2.50 g, 20.30 mmol) wurde in wässriger HCl (1 M, 40.0 ml) gelöst und auf 0°C gekühlt. Im Anschluss wurde diese Lösung über einen Zeitraum von 30 min tropfenweise mit einer Lösung aus Natriumnitrit (4.20 g, 60.90 mmol) in 120 ml H₂O versetzt, woraufhin sich das Reaktionsgemisch rotbraun färbte. Anschließend wurde das Reaktionsgemisch unter fortlaufender Kühlung über einen Zeitraum von 30 min mit einer Lösung aus KCN (3.97 g, 60.90 mmol) in 60.0 ml H₂O versetzt, wobei ein orange-gelber Feststoff ausfiel, welcher abfiltriert, einmal mit 1M HCl und zweimal mit H₂O gewaschen wurde. Der Feststoff wurde in Ethylacetat gelöst und die organische Phase jeweils einmal mit Wasser und mit gesättigter NaCl-Lösung gewaschen. Nach Trocknen der organischen Phase über Na₂SO₄ und Entfernen des Lösungsmittels unter vermindertem Druck wurde der Rückstand säulenchromatographisch an Kieselgel gereinigt (Eluent: *n-*Hexan/CH₂Cl₂/EtOAc = 80:10:10), wobei 850.0 mg (26%) der Titelverbindung als orangebraune Kristalle erhalten wurden.

### Synthese von 4-Methoxy-benzoldiazo-bis(2-hydroxyethyl)-carbamid (17)

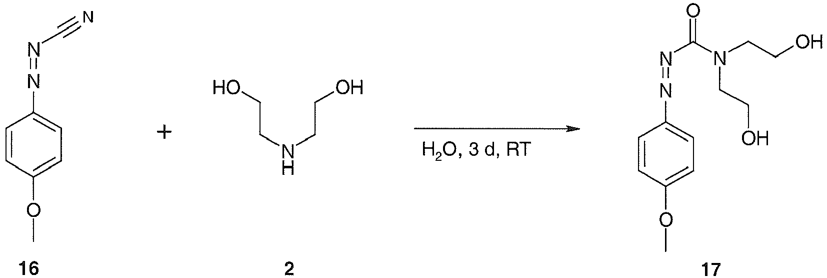

4-Methoxy-benzoldiazocyanid **(16)** (100.0 mg, 0.621 mmol) wurde in einem Gemisch aus 2.00 ml H₂O und 4.00 ml MeOH suspendiert. Im Anschluss wurde die Suspension mit Diethanolamin **(2)** (297 µl, 3.103 mmol) versetzt und das Reaktionsgemisch für 72 h bei Raumtemperatur gerührt. Nach Entfernen des Lösungsmittels unter vermindertem Druck wurde der Rückstand säulenchromatographisch an Kieselgel gereinigt (Eluent: CH₂Cl₂/MeOH = 92:8), wobei 82.3 mg (49%) der Titelverbindung als oranger Feststoff erhalten wurden.

### Synthese von 2-[(2-Hydroxyethy)-(2-methoxy-4-nitrophenyl)-amino]-ethanol (19)

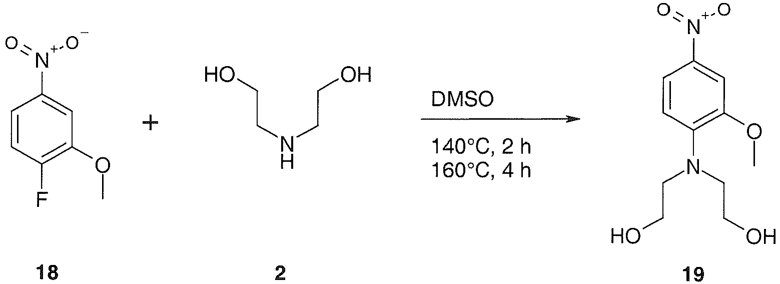

2-Fluor-5-nitroanisol **(18)** (2.00 g, 8.62 mmol) und Diethanolamin **(2)** (2.48 ml, 28.48 mmol) wurden in 20.0 ml DMSO gelöst und 2 h bei 140 °C sowie 4 h bei 160 °C refluxiert. Im Anschluss wurde das Lösungsmittel unter vermindertem Druck (3 mbar, 80 °C) entfernt, wobei ein rot-braunes Öl zurückblieb, welches in einem Gemisch aus CH₂Cl₂ und H₂O gelöst wurde. Die wässrige Phase wurde mit 2 M HCl auf pH 1 angesäuert und mehrfach mit CH₂Cl₂ extrahiert. Nach Filtration wurde die wässrige Phase mit 2 M NaOH auf pH 12 eingestellt und erneut mit CH₂Cl₂ extrahiert. Die vereinigten organischen Phasen wurden über Na₂SO₄ getrocknet und das Lösungsmittel unter vermindertem Druck entfernt, wobei ein orangefarbenes Öl erhalten wurde. Eine chromatographische Reinigung des Rückstandes an Kieselgel (Eluent: EtOAc/MeOH = 95:5) lieferte 136.5 mg (6%) der Titelverbindung.

### Synthese von 2-[(4-Amino-2-methoxyphenyl)-2-(2-hydroxyethyl)-amino]-ethanol Dihydrochlorid (20)

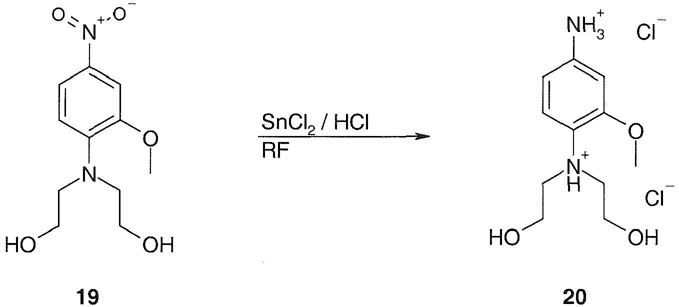

2-[(2-Hydroxyethyl)-(2-methoxy-4-nitrophenyl)-amino]-ethanol **(19)** (136.9 mg, 0.534 mmol) wurde in 10.0 ml konz. HCl (37%) gelöst, mit Zinn (II)-chlorid Dihydrat (723.2 mg, 3.205 mmol) versetzt und für 2 h bei 110°C refluxiert. Im Anschluss wurde das Gemisch durch Zugabe von Ammoniaklösung (25% in H₂O) unter Eiskühlung auf pH 8.6 eingestellt, wobei ein farbloser Feststoff ausfiel. Nach mehrfacher Extraktion der wässrigen Suspension mit Ethylacetat wurden die vereinigten organischen Phasen über Na₂SO₄ getrocknet, auf ein Viertel des ursprünglichen Volumens eingeengt, und mit 1.00 ml konz. HCl (37%) versetzt. Nach Entfernen des Lösungsmittels unter vermindertem Druck und Trocknen des Rückstands im Hochvakuum wurden 114.6 mg (71%) der Titelverbindung **(20)** erhalten.

### Synthese von 4-[Bis-(2-hydroxyethyl)-amino]-2-methoxy-benzoldiazocyanid (22)

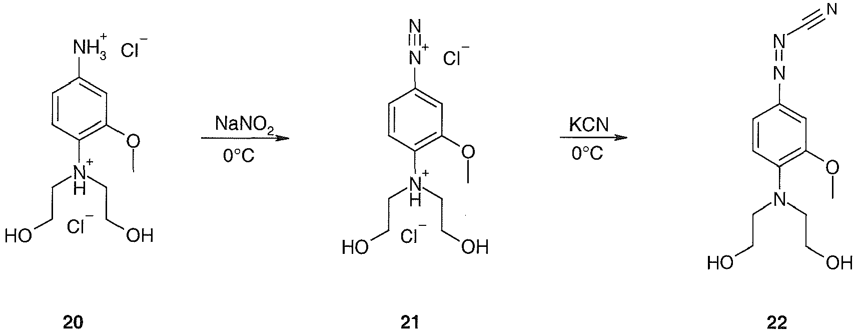

2-[(4-Amino-2-methoxyphenyl)-2-(2-hydroxyethyl)-amino]-ethanol Dihydrochlorid **(20)** (55.6 mg, 0.186 mmol) wurde in 1.80 ml H₂O gelöst und auf 0 °C gekühlt. Diese Lösung wurde über einen Zeitraum von 30 min tropfenweise mit einer Lösung aus Natriumnitrit (38.5 mg, 0.558 mmol) in 1.90 ml H₂O versetzt, woraufhin sich das Reaktionsgemisch braun färbte. Anschließend wurde dieses unter Kühlung über einen Zeitraum von 30 min tropfenweise mit einer Lösung aus KCN (36.3 mg, 0.558 mmol) in 0.60 ml H₂O versetzt, wobei eine Farbänderung nach tiefrot erfolgte. Nach 45 min Rühren bei dieser Temperatur wurde das Reaktionsgemisch mehrfach mit Ethylacetat extrahiert. Die vereinigten organischen Phasen wurden über Na₂SO₄ getrocknet und das Lösungsmittel unter vermindertem Druck entfernt. Die chromatographische Reinigung des Rückstandes an Kieselgel (Eluent: CH₂Cl₂/MeOH = 90:10) lieferte 29.7 mg (60%) der Titelverbindung als dunkelrot-violette Kristalle.

### Beispiel 2: Bestimmung des Redoxverhaltens erfindungsgemäßer Azoverbindungen

Im Rahmen der Evaluierung, inwieweit Azoverbindungen der allgemeinen Formel (I) als Mediator in diagnostischen Verfahren einsetzbar sind, wurde das Redoxverhalten eines typischen Vertreters der erfindungsgemäßen Azoverbindungen untersucht.

Zu diesem Zweck wurde zunächst eine Lösung von 4-[Bis-(2-hydroxyethyl)-amino]-benzoldiazocarbamid **(7)** (5 mM in 0.2M Kaliumphosphat-Puffer pH 7.0, 0.9% NaCl, 0.5% Triton X-100) hergestellt. Nach Zugabe von FAD-abhängiger Glucose-Dehydrogenase (20 mg/ml) wurden 40 µl dieser Lösung mit 10 µl einer Glucoselösung (Konzentration: 0 mg/dl, 100 mg/dl, 200 mg/dl, 300 mg/dl, 400 mg/dl bzw. 500 mg/dl) versetzt und für 5 min vorinkubiert.

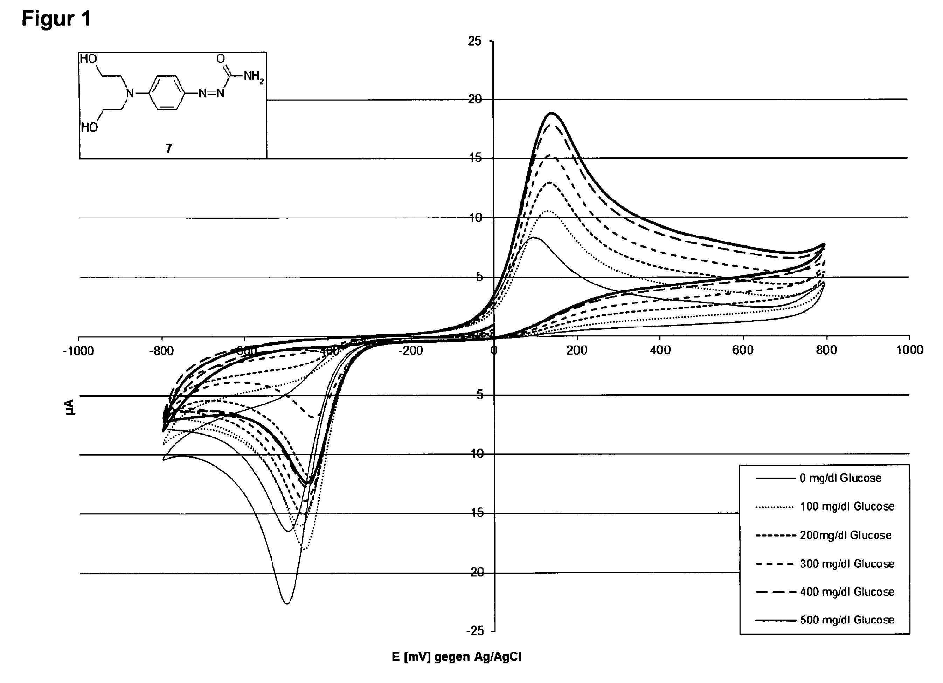

Anschließend wurde ein Cyclovoltagramm gegen Ag/AgCl aufgezeichnet. Hierzu wurde das Potential zunächst kontinuierlich von 0 mV bis -800 mV, anschließend zurück auf + 800 mV, und abschließend auf -100 mV gefahren und die hierbei fließenden Ströme gemessen. Die Spannungsänderung pro Zeiteinheit betrug 100 mV/sec. Die Ergebnisse dieser Messungen sind in Figur 1 dargestellt.

Wie aus Figur 1 hervorgeht, zeigt das Cyclovoltagramm eine deutliche Abstufung der Größe des Oxidationspeaks im Bereich zwischen 100 und 200 mV (Reoxidation des reduzierten Mediators) in Abhängigkeit von der jeweiligen Glucosekonzentration. Diese Ergebnisse weisen auf die Eignung solcher Substanzen als Mediatoren bei amperometrischen bzw. biamperometrischen Glucosebestimmungen hin.

### Beispiel 3: Kinetik der Umsetzung von Glucose unter Verwendung erfindungsgemäßer Azoverbindungen

In einem weiteren Experiment zur Evaluierung des Redoxverhaltens von Azoverbindungen der allgemeinen Formel (I) wurde beispielhaft die Kinetik der Umsetzung unterschiedlich konzentrierter Glucoselösungen in Gegenwart von NAD-abhängiger Glucose-Dehydrogenase, carbaNAD und 4-[Bis-(2-hydroxyethyl)-amino]-benzoldiazocarbamid **(7)** untersucht.

Zu diesem Zweck wurde zunächst eine Lösung von 4-[Bis-(2-hydroxyethyl)-amino]-benzoldiazocarbamid **(7)** und carbaNAD (jeweils 25 mM in 0.2M Kaliumphosphat-Puffer pH 7.0, 0.9% NaCl, 0.1% Triton X-100) hergestellt. Nach Zugabe von NAD-abhängiger Glucose-Dehydrogenase (20 mg/ml) wurden jeweils 40 µl dieser Lösung mit 10 µl einer Glucoselösung (Konzentration: 0 mg/dl, 100 mg/dl, 200 mg/dl, 300 mg/dl, 400 mg/dl bzw. 500 mg/dl) versetzt, wobei es durch die Enzym/Glucose-Reaktion zur Bildung von carbaNADH kommt, welches seinerseits eine Reduktion des Mediators bewirkt.

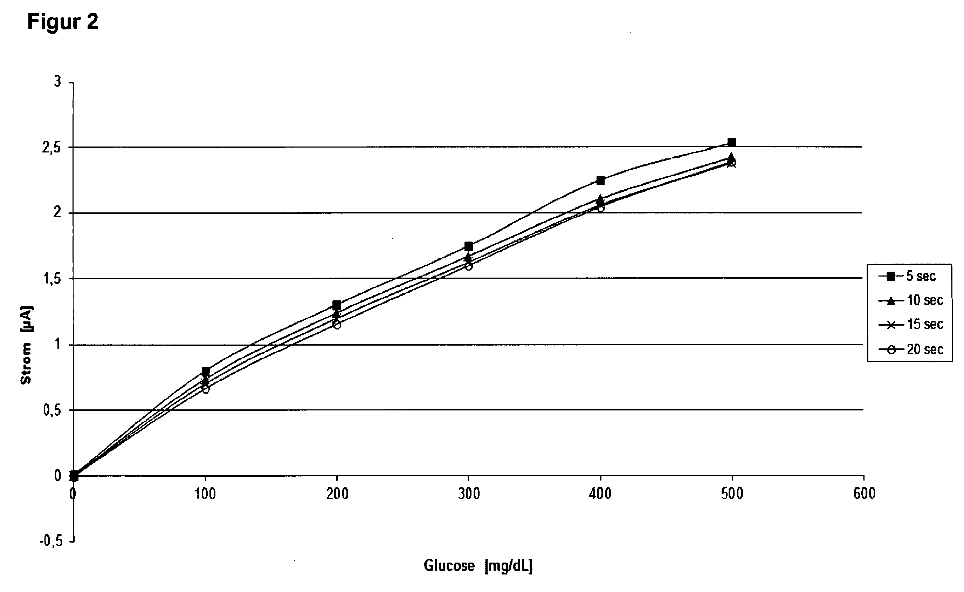

Die Kinetik der Umsetzung wurde unmittelbar nach Zugabe der Glucose (d.h. ohne Vorinkubation) amperometrisch bestimmt. Hierzu wurde ein Potential von 200 mV angelegt und die durch die Reoxidation des Mediators fließenden Ströme nach einem Zeitraum von 5, 10, 15 bzw. 20 Sekunden aufgezeichnet. Die Ergebnisse der Messungen sind in Figur 2 dargestellt.

Die annähernd lineare Korrelation zwischen der Glucosekonzentration und dem jeweils gemessenen Strom ist bereits bei kurzen Reaktionszeiten, wie beispielsweise bei einer Reaktionszeit von 5 Sekunden, klar erkennbar. Somit kann die aufgenommene Kurve als Eichgerade für amperometrische Glucosebestimmungen verwendet werden, was die in Beispiel 2 erhaltenen Ergebnisse unterstreicht.

### Beispiel 4: Kinetik der Reduktion erfindungsgemäßer Azoverbindungen

In einem weiteren Experiment zur Evaluierung des Redoxverhaltens von Azoverbindungen der allgemeinen Formel (I) wurde die Kinetik der Reduktion von 4-[Bis-(2-hydroxyethyl)-amino]-benzoldiazocarbamid (7) mit drei unterschiedlichen Reduktionsmitteln (RM) untersucht.

Zu diesem Zweck wurde 1.00 ml einer Lösung des Acetatsalzes von 4-[Bis-(2-hydroxyethyl)-amino]-benzoldiazocarbamid **(7)** (0.05 mM in 0.1M Triethylammoniumacetat-Puffer pH 7) in einer Quarzküvette jeweils mit 15 Äquivalenten des Reduktionsmittels [Ascorbinsäure; carbaNADH (als Dinatriumsalz) oder NADH (als Dinatriumsalz); jeweils als Lösung in 0.1 M Triethylammoniumacetat] umgesetzt. Die einzelnen Reaktionsbedingungen sind in Tabelle 1 zusammengefasst.

**Tabelle 1**

| **#** | **Mediator** | **Reduktionsmittel (RM)** | **Konz. RM-Lsg.** | **Vol. RM-Lsg.** | **Äq. RM** |
|---|---|---|---|---|---|
| 1 | **7** | Ascorbinsäure Na₂ | 0.1M | 7.5 µl | 15 |
| 2 | **7** | carbaNADH Na₂ | 0.005M | 150 µl | 15 |
| 3 | **7** | NADH Na₂ | 0.05M | 15 µl | 15 |

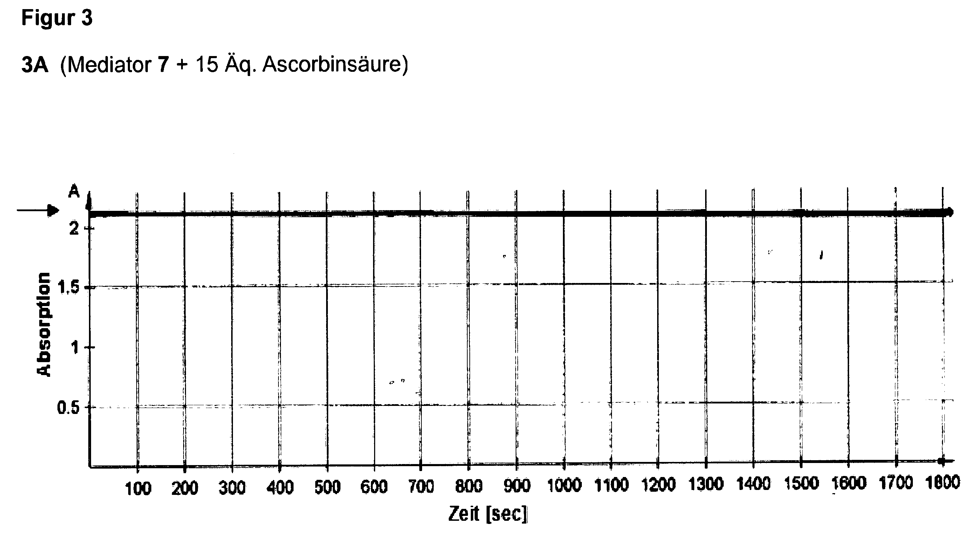

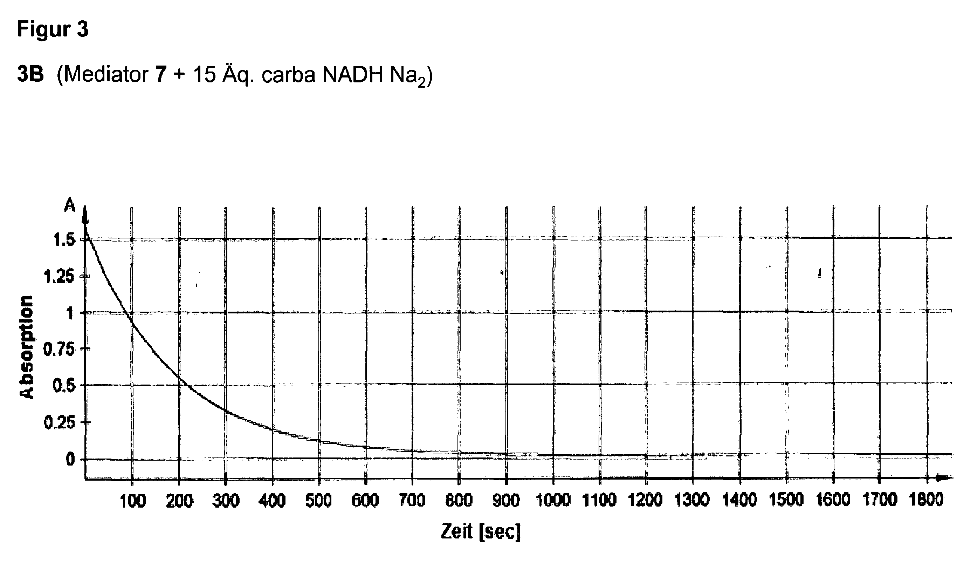

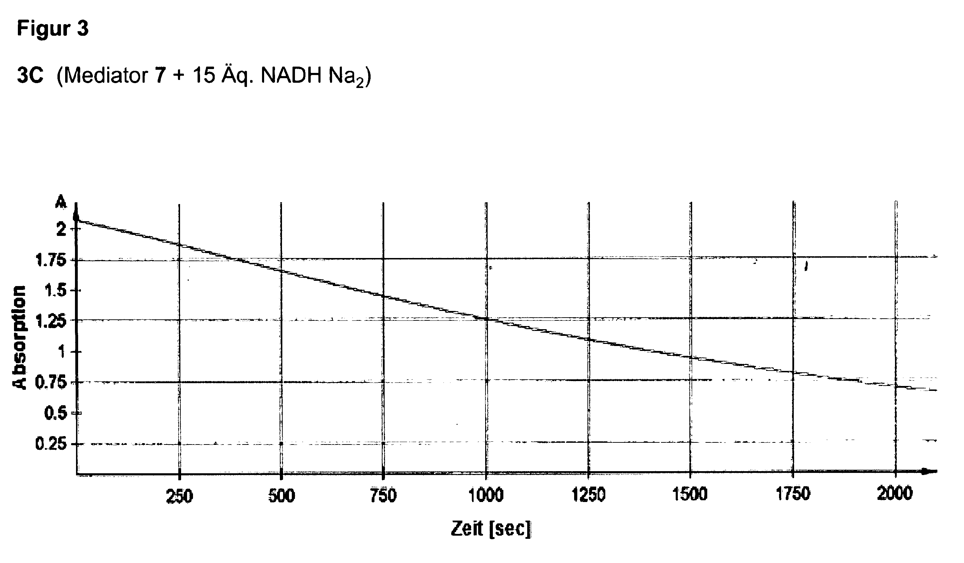

Die Kinetik der erwarteten Reduktion von 4-[Bis-(2-hydroxyethyl)-amino]-benzoldiazocarbamid **(7)** wurde unmittelbar nach Zugabe des Reduktionsmittels mittels UV-Spektroskopie anhand der Abnahme der Absorption bei 500 nm (Absorptionsmaximum der oxidierten Form des eingesetzten Mediators) aufgezeichnet (UV-Spektrometer: SPECORD 210, Software: WinASPECT Version 2.2.2.0, Analytik Jena AG). Die Ergebnisse dieser Messungen sind in den Figuren 3A, 3B und 3C dargestellt. Figur 3A zeigt den Reaktionsverlauf in Gegenwart von Ascorbinsäure und verdeutlicht die Unempfindlichkeit von 4-[Bis-(2-hydroxyethyl)-amino]-benzoldiazocarbamid **(7)** gegenüber diesem Reduktionsmittel. Tatsächlich war im zeitlichen Verlauf auch nach 1800 Sekunden keine merkliche Abnahme der Menge an Mediator (oxidierte Form) zu verzeichnen. Die Zugabe eines noch größeren Überschusses an Ascorbinsäure nach dem Messzeitraum führte zum selben Ergebnis.

Die Figuren 3B und 3C zeigen den Reaktionsverlauf der Reduktion von 4-[Bis-(2-hydroxyethyl)-amino]-benzoldiazocarbamid **(7)** mittels carbaNADH bzw. NADH. Wie aus den Figuren hervorgeht, bewirkt sowohl carbaNADH als auch NADH eine Reduktion des Mediators, wobei sich aus dem Vergleich beider Kurven eine wesentlich höhere Reduktionsgeschwindigkeit für das artifizielle Coenzym carbaNADH ableiten lässt.

## Patentansprüche

1. Azoverbindung der allgemeinen Formel (I) worin 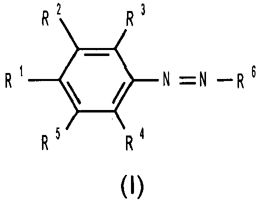
R¹ einen Rest ausgewählt aus der Gruppe bestehend aus OR⁷ und NR⁷R⁸ bedeutet;
R², R³, R⁴ und R⁵ unabhängig voneinander einen Rest ausgewählt aus der Gruppe bestehend aus H, Alkyl, Cycloalkyl, Alkenyl, Alkinyl, Aryl, Heterocycloalkyl, Heteroaryl, CN, COO⁻, Halogen, O(Alkyl), O(Aryl), NH(Alkyl), N(Alkyl)₂, N(Alkyl)₃⁺, NH(Aryl), N(Aryl)₂, NO₂ und SO₃⁻ bedeuten, wobei das Alkyl, Cycloalkyl, Alkenyl, Alkinyl, Aryl, Heterocycloalkyl, Heteroaryl, O(Alkyl), O(Aryl), NH(Alkyl), N(Alkyl)₂, N(Alkyl)₃⁺, NH(Aryl) oder N(Aryl)₂ einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)n-O(Alkyl), OPO₃²⁻, PO₃²⁻ und SO₃⁻ umfassen kann;
R⁶ einen Rest ausgewählt aus der Gruppe bestehend aus C(=X)NR⁹R¹⁰, C(=X)NHOH und C(=X)R⁹ bedeutet;
R⁷ einen Rest ausgewählt aus der Gruppe bestehend aus Alkyl, Cycloalkyl, Alkenyl, Alkinyl, Aryl, Heterocycloalkyl und Heteroaryl bedeutet, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst,
R⁸ einen Rest ausgewählt aus der Gruppe bestehend aus Alkyl, Cycloalkyl, Alkenyl, Alkinyl, Aryl, Heterocycloalkyl und Heteroaryl bedeutet, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfassen kann, oder
R⁷ und R⁸ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N,
O und S umfassen kann und einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst;
R⁹ und R¹⁰ unabhängig voneinander einen Rest ausgewählt aus der Gruppe bestehend aus Alkyl, Cycloalkyl, Alkenyl, Alkinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O(Alkyl), O-(CH₂CH₂)ₙ-OH, O-(CH₂CH₂)ₙ-O(Alkyl), O(Aralkyl), O(Aryl), OPO₃²⁻, PO₃²⁻ und SO₃⁻ umfassen kann, oder
R⁹ und R¹⁰ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O(Alkyl), O-(CH₂CH₂)ₙ-OH, O-(CH₂CH₂)ₙ-O(Alkyl), O(Aralkyl), O(Aryl), OPO₃²⁻, PO₃²⁻ und SO₃⁻ umfassen kann;
X ein Heteroatom ausgewählt aus der Gruppe bestehend aus O und S bedeutet; und
n eine ganze Zahl von 1 bis 6 bedeutet;
mit der Maßgabe, dass mindestens zwei Reste ausgewählt aus der Gruppe bestehend aus R², R³, R⁴ und R⁵ den Rest H bedeuten und die Azoverbindung der allgemeinen Formel (I) nicht 2-(Ethyl-{3-methyl-4-[(piperidin-1-ylcarbonyl)diazenyl]phenyl}amino)ethanol ist.

2. Azoverbindung nach Anspruch 1,
**dadurch gekennzeichnet,**
**dass** R¹ den Rest NR⁷R⁸ bedeutet.

3. Azoverbindung nach Anspruch 1 oder 2,
**dadurch gekennzeichnet,**
**dass** mindestens drei Reste ausgewählt aus der Gruppe bestehend aus R², R³, R⁴ und R⁵ den Rest H bedeuten.

4. Azoverbindung nach einem der Ansprüche 1 bis 3,
**dadurch gekennzeichnet,**
**dass** R² einen Rest ausgewählt aus der Gruppe bestehend aus H und O(Alkyl) bedeutet, und R³, R⁴ und R⁵ jeweils den Rest H bedeuten.

5. Azoverbindung nach einem der Ansprüche 1 bis 4,
**dadurch gekennzeichnet,**
**dass** R⁶ den Rest C(=X)NR⁹R¹⁰, insbesondere den Rest C(=O)NR⁹R¹⁰, bedeutet.

6. Azoverbindung nach einem der Ansprüche 1 bis 5,
**dadurch gekennzeichnet,**
**dass** R⁸ einen oder mehrere Substituenten ausgewählt aus der Gruppe bestehend aus COO⁻, N(Alkyl)₃⁺, NH-C(=NH₂)-NH₂⁺, OH, O-(CH₂CH₂)ₙ-OH, O-(CH₂CH₂)ₙ-O(Alkyl), OPO₃²⁻, PO₃²⁻ und SO₃⁻ umfasst.

7. Azoverbindung nach einem der Ansprüche 1 bis 6,
**dadurch gekennzeichnet,**
**dass** R⁷ oder/und R⁸ den Rest Hydroxyethyl bedeutet.

8. Azoverbindung nach einem der Ansprüche 1 bis 7,
**dadurch gekennzeichnet,**
**dass** R⁹ oder/und R¹⁰ einen Rest ausgewählt aus der Gruppe bestehend aus Alkyl und Aryl bedeutet, wobei das Alkyl oder Aryl einen oder mehrere Substituenten ausgewählt aus der Gruppe bestehend aus OH, O(Alkyl) und O(Aryl) umfassen kann, oder
R⁹ und R¹⁰ zusammen mit dem N-Atom, an welches sie gebunden sind, einen 5- oder 6-gliedrigen Heterocyclus bilden, wobei der Heterocyclus ein weiteres Heteroatom ausgewählt aus der Gruppe bestehend aus N, O und S umfassen kann.

9. Azoverbindung nach einem der Ansprüche 1 bis 8,
**dadurch gekennzeichnet,**
**dass** R⁹ oder/und R¹⁰ einen Rest ausgewählt aus der Gruppe bestehend aus Methyl, Ethyl, Phenyl, Hydroxymethyl, Hydroxyethyl, Hydroxyphenyl und Methoxyphenyl bedeutet.

10. Verwendung einer Azoverbindung der allgemeinen Formel (I) 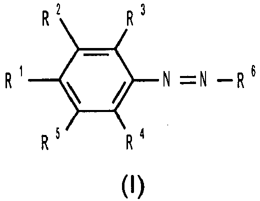 als Mediator in einem diagnostischen Verfahren, worin
R¹ einen Rest ausgewählt aus der Gruppe bestehend aus OH, OR⁷, SR⁷, NHR⁷ und NR⁷R⁸ bedeutet;
R^{z}, R³, R⁴ und R⁵ unabhängig voneinander einen Rest ausgewählt aus der Gruppe bestehend aus H, Alkyl, Cycloalkyl, Alkenyl, Alkinyl, Aryl, Heterocycloalkyl, Heteroaryl, CN, COO⁻, Halogen, O(Alkyl), O(Aryl), NH(Alkyl), N(Alkyl)₂, N(Alkyl)₃⁺, NH(Aryl), N(Aryl)₂, NO₂ und SO₃⁻ bedeuten, wobei das Alkyl, Cycloalkyl, Alkenyl, Alkinyl, Aryl, Heterocycloalkyl, Heteroaryl, O(Alkyl), O(Aryl), NH(Alkyl), N(Alkyl)₂, N(Alkyl)₃⁺, NH(Aryl) oder N(Aryl)₂ einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann;
R⁶ einen Rest ausgewählt aus der Gruppe bestehend aus CN, C(=X)NH₂, C(=_{X})NHR⁹, C(=X)NR⁹R¹⁰, C(=X)NHOH und C(=X)R⁹ bedeutet;
R⁷ und R⁸ unabhängig voneinander einen Rest ausgewählt aus der Gruppe bestehend aus Alkyl, Cycloalkyl, Alkenyl, Alkinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann, oder
R⁷ und R⁸ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²- und SO₃⁻ umfassen kann;
R⁹ und R¹⁰ unabhängig voneinander einen Rest ausgewählt aus der Gruppe bestehend aus Alkyl, Cycloalkyl, Alkenyl, Alkinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann, oder
R⁹ und R¹⁰ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann;
X ein Heteroatom ausgewählt aus der Gruppe bestehend aus O und S bedeutet; und
n eine ganze Zahl von 1 bis 6 bedeutet.

11. Nachweisreagenz zur Bestimmung eines Analyten, umfassend
(a) eine Flavin-, Nicotinamid- oder Pyrrolochinolinchinon-abhängige Oxidoreduktase,
(b) ein reduzierbares Flavin-, Nicotinamid- oder Pyrrolochinolinchinon-Coenzym,
(c) eine Azoverbindung der allgemeinen Formel (I) worin 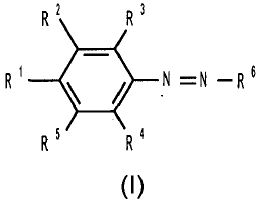
R¹ einen Rest ausgewählt aus der Gruppe bestehend aus OH, OR⁷, SR⁷, NHR⁷ und NR⁷R⁸ bedeutet;
R², R³, R⁴ und R⁵ unabhängig voneinander einen Rest ausgewählt aus der Gruppe bestehend aus H, Alkyl, Cycloalkyl, Alkenyl, Alkinyl, Aryl, Heterocycloalkyl, Heteroaryl, CN, COO⁻, Halogen, O(Alkyl), O(Aryl), NH(Alkyl), N(Alkyl)₂, N(Alkyl)₃⁺, NH(Aryl), N(Aryl)₂, NO₂ und SO₃⁻ bedeuten, wobei das Alkyl, Cycloalkyl, Alkenyl, Alkinyl, Aryl, Heterocycloalkyl, Heteroaryl, O(Alkyl), O(Aryl), NH(Alkyl), N(Alkyl)₂, N(Alkyl)₃⁺, NH(Aryl) oder N(Aryl)₂ einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann;
R⁶ einen Rest ausgewählt aus der Gruppe bestehend aus CN, C(=X)NH₂, C(=X)NHR⁹, C(=X)NR⁹R¹⁰, C(=X)NHOH und C(=X)R⁹ bedeutet;
R⁷ und R⁸ unabhängig voneinander einen Rest ausgewählt aus der Gruppe bestehend aus Alkyl, Cycloalkyl, Alkenyl, Alkinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann, oder
R⁷ und R⁸ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann;
R⁹ und R¹⁰ unabhängig voneinander einen Rest ausgewählt aus der Gruppe bestehend aus Alkyl, Cycloalkyl, Alkenyl, Alkinyl, Aryl, Heterocycloalkyl und Heteroaryl bedeuten, wobei das Alkyl, Cycloalkyl, Alkenyl, Alkinyl, Aryl, Heterocycloalkyl oder Heteroaryl einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃⁻ umfassen kann, oder
R⁹ und R¹⁰ zusammen mit dem N-Atom, an welches sie gebunden sind, einen 5- bis 7-gliedrigen Heterocyclus bilden, wobei der Heterocyclus ein weiteres Heteroatom ausgewählt aus der Gruppe bestehend aus N, O und S oder/und einen oder mehrere Substituenten ausgewählt aus der Gruppe bestehend aus CN, COO⁻, Halogen, NH₂, NH(Alkyl), N(Alkyl)₂, N(Alkyl)₃⁺, NH(Aryl), N(Aryl)₂, NH-C(=NH₂)-NH₂⁺, NO₂, OH, O-(CH₂CH₂)ₙ-OH, O-(CH₂CH₂)ₙ-O(Alkyl), O(Alkyl), O(Aralkyl), O(Aryl), OPO₃²⁻, PO₃²⁻ und SO₃ umfassen kann;
X ein Heteroatom ausgewählt aus der Gruppe bestehend aus O und S bedeutet; und
n eine ganze Zahl von 1 bis 6 bedeutet, und
(d) gegebenenfalls einen reduzierbaren optischen Indikator.

12. Nachweisreagenz nach Anspruch 11,
**dadurch gekennzeichnet,**
**dass** die Flavin-, Nicotinamid- oder Pyrrolochinolinchinon-abhängige Oxidoreduktase eine Flavin-, Nicotinamid- oder Pyrrolochinolinchinon-abhängige Dehydrogenase, insbesondere eine Flavin-, Nicotinamid- oder Pyrrolochinolinchinon-abhängige Glucose-Dehydrogenase oder Glucose-6-phosphat-Dehydrogenase, ist,
und/oder
**dass** das reduzierbare Flavin-, Nicotinamid- oder Pyrrolochinolinchinon-Coenzym FAD, FMN, NAD⁺, NADP⁺, PQQ, oder ein Derivat hiervon, insbesondere NAD⁺, NADP⁺, carbaNAD⁺ oder carbaNADP⁺, ist.

13. Kit zur Bestimmung eines Analyten, umfassend
(a) ein Nachweisreagenz nach einem der Ansprüche 11 bis 12, und
(b) ein Testelement, insbesondere ein als optischer oder elektrochemischer Sensor ausgebildetes Testelement.

14. Testelement zur Bestimmung eines Analyten, umfassend ein Nachweisreagenz nach einem der Ansprüche 11 bis 12, wobei das Testelement vorzugsweise als optischer oder elektrochemischer Sensor ausgebildet ist.

15. Verfahren zur Bestimmung eines Analyten, umfassend die Schritte:
(a) Inkontaktbringen einer den Analyten enthaltenden Probe mit einem Nachweisreagenz nach einem der Ansprüche 11 bis 12, einem Kit nach Anspruch 13, oder einem Testelement nach Anspruch 14, und
(b) Bestimmen des Vorhandenseins oder/und der Menge des Analyten.

## Claims

1. Azo compound having the general formula (I) 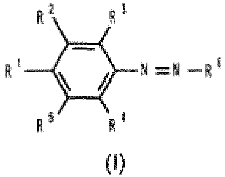 in which
R¹ is a residue selected from the group comprising OR⁷ and NR⁷R⁸,
R², R³, R⁴ and R⁵ are, independently of one another, each a residue selected from the group comprising H, alkyl, cycloalkyl, alkenyl, alkinyl, aryl, heterocycloalkyl, heteroaryl, CN, COO⁻, halogen, O(alkyl), O(aryl), NH(alkyl), N(alkyl)₂, N(alkyl)₃⁺, NH(aryl), N(aryl)₂, NO₂ and SO₃⁻, in which case the alkyl, cycloalkyl, alkenyl, alkinyl, aryl, heterocycloalkyl, heteroaryl, O(alkyl), O(aryl), NH(alkyl), N(alkyl)₂, N(alkyl)₃⁺, NH(aryl) or N(aryl)₂ may comprise one or more substituents selected from the group comprising COO⁻, N(alkyl)₃⁺, NH-C(=NH₂)-NH₂⁺, OH, O-(CH₂CH₂)ₙ-OH, O-(CH₂-CH₂)ₙ-O(alkyl), OPO₃²⁻, PO₃²⁻ and SO₃⁻,
R⁶ is a residue selected from the group comprising C(=X)NR⁹R¹⁰, C(=X)NHOH and C(=X)R⁹,
R⁷ is a residue selected from the group comprising alkyl, cycloalkyl, alkenyl, alkinyl, aryl, heterocycloalkyl and heteroaryl, in which case the alkyl, cycloalkyl, alkenyl, alkinyl, aryl, heterocycloalkyl or heteroaryl comprises one or more substituents selected from the group comprising COO⁻, N(alkyl)₃⁺, NH-C(=NH₂)-NH₂⁺, OH, O-(CH₂CH₂)ₙ-OH, O-(CH₂-CH₂)ₙ-O(alkyl), OPO₃²⁻, PO₃²⁻ and SO₃⁻,
R⁸ is a residue selected from the group comprising alkyl, cycloalkyl, alkenyl, alkinyl, aryl, heterocycloalkyl and heteroaryl, in which case the alkyl, cycloalkyl, alkenyl, alkinyl, aryl, heterocycloalkyl or heteroaryl may comprise one or more substituents selected from the group comprising COO⁻, N(alkyl)₃⁺, NH-C(=NH₂)-NH₂⁺, OH, O-(CH₂CH₂)ₙ-OH, O-(CH₂-CH₂)ₙ-O (alkyl), OPO₃²⁻, PO₃²⁻ and SO₃⁻, or
R⁷ and R⁸, together with the N atom to which they are bonded, form a 5 to 7-membered heterocycle, in which case the heterocycle may comprise a further heteroatom selected from the group comprising N, 0 and S and comprises one or more substituents selected from the group comprising COO⁻, N(alkyl)₃⁺, NH-C(=NH₂)-NH₂⁺, OH, O-(CH₂CH₂)ₙ-OH, O-(CH₂-CH₂)ₙ-O(alkyl), OPO₃²⁻, PO₃²⁻ and SO₃⁻,
R⁹ and R¹⁰ are, independently of one another, each a residue selected from the group comprising alkyl, cycloalkyl, alkenyl, alkinyl, aryl, heterocycloalkyl and heteroaryl, in which case the alkyl, cycloalkyl, alkenyl, alkinyl, aryl, heterocycloalkyl or heteroaryl may comprise one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O(alkyl), O-(CH₂CH₂)ₙ-OH, O-(CH₂-CH₂)ₙ-O(alkyl), O(aralkyl), O(aryl), OPO₃²⁻, PO₃²⁻ and SO₃⁻, or
R⁹ and R¹⁰, together with the N atom to which they are bonded, form a 5 to 7-membered heterocycle, in which case the heterocycle may comprise a further heteroatom selected from the group comprising N, 0 and S or/and one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O(alkyl), O-(CH₂CH₂)ₙ-OH, O-(CH₂-CH₂)ₙ-O (alkyl), O(aralkyl), O(aryl), OPO₃²⁻, PO₃²⁻ and SO₃⁻,
X is a heteroatom selected from the group comprising 0 and S, and
n is an integer from 1 to 6,
with the proviso that at least two residues selected from the group comprising R², R³, R⁴ and R⁵ are the residue H and the azo compound having the general formula (I) is not 2-(ethyl-{3-methyl-4-[(piperidin-lylcarbonyl)diazenyl]phenyl} amino)ethanol.

2. Azo compound according to claim 1, **characterised in that** R¹ is the residue NR⁷R⁸.

3. Azo compound according to claim 1 or 2, **characterised in that** at least three residues selected from the group comprising R², R³, R⁴ and R⁵ are the residue H.

4. Azo compound according to one of claims 1 to 3, **characterised in that** R² is a residue selected from the group comprising H and O(alkyl) and R³, R⁴ and R⁵ are each the residue H.

5. Azo compound according to one of claims 1 to 4, **characterised in that** R⁶ is the residue C(=X)NR⁹R¹⁰, and in particular the residue C(=O)NR⁹R¹⁰.

6. Azo compound according to one of claims 1 to 5, **characterised in that** R⁸ comprises one or more substituents selected from the group comprising COO⁻, N(alkyl)₃⁺, NH-C(=NH2)-NH₂⁺, OH, O-(CH₂CH₂)ₙ-OH, O-(CH₂-CH₂)ₙ-O(alkyl), OPO₃²⁻, PO₃²⁻ and SO₃⁻.

7. Azo compound according to one of claims 1 to 6, **characterised in that** R⁷ or/and R⁸ is the residue hydroxyethyl.

8. Azo compound according to one of claims 1 to 7, **characterised in that** R⁹ or/and R¹⁰ is a residue selected from the group comprising alkyl and aryl, in which case the alkyl or aryl may comprise one or more substituents selected from the group comprising OH, O(alkyl) and O(aryl), or
R⁹ and R¹⁰, together with the N atom to which they are bonded, form a 5 or 6-membered heterocycle, in which case the heterocycle may comprise a further heteroatom selected from the group comprising N, 0 and S.

9. Azo compound according to one of claims 1 to 8, **characterised in that** R⁹ or/and R¹⁰ is a residue selected from the group comprising methyl, ethyl, phenyl, hydroxymethyl, hydroxyethyl, hydroxyphenyl and methoxyphenyl.

10. Use of an azo compound having the general formula (I) 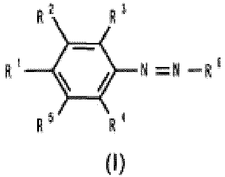 as a mediator in a diagnostic process, in which R¹ is a residue selected from the group comprising OH, OR⁷, SR⁷, NHR⁷ and NR⁷R⁸,
R², R³, R⁴ and R⁵ are, independently of one another, each a residue selected from the group comprising H, alkyl, cycloalkyl, alkenyl, alkinyl, aryl, heterocycloalkyl, heteroaryl, CN, COO⁻, halogen, O(alkyl), O(aryl), NH(alkyl), N(alkyl)₂, N(alkyl)₃⁺, NH(aryl), N(aryl)₂, NO₂ and SO₃⁻, in which case the alkyl, cycloalkyl, alkenyl, alkinyl, aryl, heterocycloalkyl, heteroaryl, O(alkyl), O(aryl), NH(alkyl), N(alkyl)₂, N(alkyl)₃⁺, NH(aryl) or N(aryl)₂ may comprise one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙ-OH, O-(CH₂-CH₂)ₙ-O O(aralkyl), O(aryl), OPO₃²⁻, PO₃²⁻ and SO₃⁻,
R⁶ is a residue selected from the group comprising CN, C(=X)NH₂, C(=X)NHR⁹, C(=X)NR⁹R¹⁰, C (=X)NHOH and C(=X)R⁹, R⁷ and R⁸ are, independently of one another, each a residue selected from the group comprising alkyl, cycloalkyl, alkenyl, alkinyl, aryl, heterocycloalkyl and heteroaryl, in which case the alkyl, cycloalkyl, alkenyl, alkinyl, aryl, heterocycloalkyl or heteroaryl may comprise one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙ-OH, O-(CH₂-CH₂)ₙ-O(alkyl), O(alkyl), O(aralkyl), O(aryl), OPO₃²⁻, PO₃²⁻ and SO₃⁻, or
R⁷ and R⁸, together with the N atom to which they are bonded, form a 5 to 7-membered heterocycle, in which case the heterocycle may comprise a further heteroatom selected from the group comprising N, 0 and S and may comprise one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙ-OH, O-(CH₂-CH₂)ₙ-O(aralkyl), O(aryl), OPO₃²⁻, PO₃²⁻ and SO₃⁻,
R⁹ and R¹⁰ are, independently of one another, each a residue selected from the group comprising alkyl, cycloalkyl, alkenyl, alkinyl, aryl, heterocycloalkyl and heteroaryl, in which case the alkyl, cycloalkyl, alkenyl, alkinyl, aryl, heterocycloalkyl or heteroaryl may comprise one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙ-OH, O-(CH₂-CH₂)ₙ-O(alkyl), O(aralkyl), O(aryl), OPO₃²⁻, PO₃²⁻ and SO₃⁻, or
R⁹ and R¹⁰, together with the N atom to which they are bonded, form a 5 to 7-membered heterocycle, in which case the heterocycle may comprise a further heteroatom selected from the group comprising N, 0 and S or/and one or more substituents selected from CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙ-OH, O-(CH₂CH₂)ₙ-O(alkyl), O(Alkyl), O(aralkyl), O(aryl), OPO₃²⁻, PO₃²⁻ and SO₃⁻,
X is a heteroatom selected from the group comprising 0 and S, and
n is an integer from 1 to 6.

11. Detection reagent for determining an analyte, comprising
(a) a flavin-, nicotinamide- or pyrroloquinoline quinone-dependent oxidoreductase,
(b) a reducible flavin, nicotinamide or pyrroloquinoline quinine co-enzyme,
(c) an azo compound having the general formula 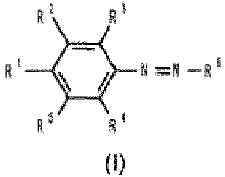 in which
R¹ is a residue selected from the group comprising OH, OR⁷, SR⁷, NHR⁷ and NR⁷R⁸,
R², R³, R⁴ and R⁵ are, independently of one another, each a residue selected from the group comprising H, alkyl, cycloalkyl, alkenyl, alkinyl, aryl, heterocycloalkyl, heteroaryl, CN, COO⁻, halogen, O(alkyl), O(aryl), NH(alkyl), N(alkyl)₂, N(alkyl) ₃⁺, NH(aryl), N(aryl)₂, NO₂ and SO₃⁻, in which case the alkyl, cycloalkyl, alkenyl, alkinyl, aryl, heterocycloalkyl, heteroaryl, O(alkyl), O(aryl), NH(alkyl), N(alkyl)₂, N(alkyl)₃⁺, NH(aryl) or N(aryl)₂ may comprise one or more substituents selected from the group comprising CN, COO⁻, halogen, NH₂, NH(alkyl), N(alkyl)₂, N(alkyl)³₊, NH(aryl), N(aryl)₂, NH-C(=NH₂)-NH₂⁺, NO₂, OH, O-(CH₂CH₂)ₙ-OH, O-(CH₂-CH₂)ₙ-O(alkyl), O(alkyl), O(aralkyl), O(aryl), OPO₃²⁻, PO₃²⁻ and SO₃⁻,
R⁶ is a residue selected from the group comprising CN, C(=X)NH₂, C(=X)NHR⁹, C(=X)NR⁹R¹⁰, C(=X)NHOH and C(=X)R⁹,
R⁷ and R⁸ are, independently of one another, each a residue selected from the group comprising alkyl, cycloalkyl, alkenyl, alkinyl, aryl, heterocycloalkyl and heteroaryl, in which case the alkyl, cycloalkyl, alkenyl, alkinyl, aryl, heterocycloalkyl or heteroaryl may comprise one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙO-OH, O-(CH₂-CH₂)ₙ-O(alkyl), O(alkyl), O(aralkyl), O(aryl), OPO₃²⁻, PO₃²⁻ and SO₃⁻, or
R⁷ and R⁸, together with the N atom to which they are bonded, form a 5 to 7-membered heterocycle, in which case the heterocycle may comprise a further heteroatom selected from the group comprising N, 0 and S or/and one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙ-OH, O-(CH₂-CH₂)ₙ-O(alkyl), O(alkyl), O(aralkyl), O(aryl), OPO₃²⁻, PO₃²⁻ and SO₃⁻,
R⁹ and R¹⁰ are, independently of one another, each a residue selected from the group comprising alkyl, cycloalkyl, alkenyl, alkinyl, aryl, heterocycloalkyl and heteroaryl, in which case the alkyl, cycloalkyl, alkenyl, alkinyl, aryl, heterocycloalkyl or heteroaryl may comprise one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙ-OH, O-(CH₂-CH₂)ₙ-O(alkyl), O(alkyl), O(aralkyl), O(aryl), OPO₃²⁻ PO₃²⁻ and SO₃⁻, or
R⁹ and R¹⁰, together with the N atom to which they are bonded, form a 5 to 7-membered heterocycle, in which case the heterocycle may comprise a further heteroatom selected from the group comprising N, 0 and S or/and one or more substituents selected from the group comprising CN, COO⁻, halogen, NH₂, NH(alkyl), N(alkyl)₂, N(alkyl)₃⁺, NH(aryl), N(aryl)₂, NH-C(=NH₂)-NH₂⁺, NO₂, OH, O-(CH₂CH₂)ₙ-OH, O-(CH₂-CH₂)ₙ-O(alkyl), O(alkyl), O(aralkyl), O(aryl), OPO₃²⁻, PO₃²⁻ and SO₃⁻,
X is a heteroatom selected from the group comprising 0 and S, and
n is an integer from 1 to 6, and
(d) if required, a reducible optical indicator.

12. Detection reagent according to claim 11, **characterised in that** the flavin-, nicotinamide- or pyrroloquinoline quinone-dependent oxidoreductase is a flavin-, nicotinamide- or pyrroloquinoline quinone-dependent dehydrogenase and in particular a flavin-, nicotinamide- or pyrroloquinoline quinone-dependent glucose dehydrogenase or glucose-6-phosphate dehydrogenase,
and/or
**in that** the reducible flavin, nicotinamide or pyrroloquinoline quinine co-enzyme is FAD, FMN, NAD⁺, NADP⁺, PQQ, or a derivate thereof and in particular NAD⁺, NADP⁺, carba-NAD⁺ or carba-NADP⁺.

13. Kit for determining an analyte, comprising
(a) a detection reagent according to either of claims 11 and 12, and
(b) a testing member and in particular a testing member in the form of an optical or electrochemical sensor.

14. Testing member for determining an analyte, comprising a detection reagent according to either of claims 11 and 12, the testing member preferably taking the form of an optical or electrochemical sensor.

15. Method of determining an analyte, comprising the steps of
(a) bringing a specimen containing the analyte into contact with a detection reagent according to either of claims 11 and 12, a kit according to claim 13, or a testing member according to claim 14, and
(b) determining the presence or/and quantity of the analyte.

## Revendications

1. Composé azo de formule générale (I) 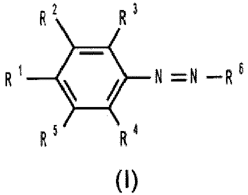 dans laquelle
R¹ représente un radical sélectionné parmi le groupe constitué de OR⁷ et NR⁷R⁸;
R², R³, R⁴ et R⁵ représentent, indépendamment les uns des autres, un radical sélectionné parmi le groupe constitué de H, alkyle, cycloalkyle, alcényle, alcinyle, aryle, hétérocycloalkyle, hétéroaryle, CN, COO⁻, halogène, O(alkyle), O(aryle), NH(alkyle), N(alkyle)₂, N(alkyle)₃⁺, NH(aryle), N(aryle)₂, NO₂, et SO₃⁻, dans lequel l'alkyle, le cycloalkyle, l'alcényle, l'alcinyle, l'aryle, l'hétérocycloalkyle, l'hétéroaryle, l'O(alkyle), l'O(aryle), le NH(alkyle), le N(alkyle)₂, le N(alkyle)₃⁺, le NH(aryle) ou le N(aryle)₂ peut comprendre un ou plusieurs substituant(s) sélectionné(s) parmi le groupe constitué de COO⁻, N(alkyle)₃⁺, NH-C(=NH₂)-NH₂⁺, OH, O-(CH₂CH₂)ₙ-OH, O-(CH₂CH₂)ₙ-O(alkyle), OPO₃²⁻, PO₃²⁻ et SO₃⁻;
R⁶ représente un radical sélectionné parmi le groupe constitué de C(=X)NR⁹R¹⁰, C(=X)NHOH et C(=X)R⁹;
R⁷ représente un radical sélectionné parmi le groupe constitué de alkyle, cycloalkyle, alcényle, alcinyle, aryle, hétérocycloalkyle et hétéroaryle, dans lequel l'alkyle, le cycloalkyle, l'alcényle, l'alcinyle, l'aryle, l'hétérocycloalkyle ou l'hétéroaryle comprennent un ou plusieurs substituant(s) sélectionné(s) parmi le groupe constitué de COO⁻, N(alkyle)₃⁺, NH-C(=NH₂)-NH₂⁺, OH, O-(CH₂CH₂)ₙ-OH, O-(CH₂CH₂)ₙ-O(alkyle), OPO₃²⁻, PO₃²⁻ et SO₃⁻;
R⁸ représente un radical sélectionné parmi le groupe constitué de alkyle, cycloalkyle, alcényle, alcinyle, aryle, hétérocycloalkyle et hétéroaryle, dans lequel l'alkyle, le cycloalkyle, l'alcényle, l'alcinyle, l'aryle, l'hétérocycloalkyle ou l'hétéroaryle peut comprendre un ou plusieurs substituant(s) sélectionné(s) parmi le groupe constitué de COO⁻, N(alkyle)₃⁺, NH-C(=NH₂)-NH₂⁺, OH, O-(CH₂CH₂)ₙ-OH, O-(CH₂CH₂)ₙ-O(alkyle), OPO₃²⁻, PO₃²⁻ et SO₃⁻, ou
R⁷ et R⁸ forment, en commun avec l'atome N auquel ils sont liés, un hétérocycle pentagonal à heptagonal, dans lequel l'hétérocycle peut comprendre un autre hétéroatome sélectionné parmi le groupe constitué de N, O et S et comprend un ou plusieurs substituant(s) sélectionné(s) parmi le groupe constitué de COO⁻, N(alkyle)₃⁺, NH-C(=NH₂)-NH₂⁺, OH, O-(CH₂CH₂)ₙ-OH, O-(CH₂CH₂)ₙ-O(alkyle), OPO₃²⁻, PO₃²⁻ et SO₃⁻;
R⁹ et R¹⁰ représentent, indépendamment l'un de l'autre, un radical sélectionné parmi le groupe constitué de alkyle, cycloalkyle, alcényle, alcinyle, aryle, hétérocycloalkyle et hétéroaryle, dans lequel l'alkyle, le cycloalkyle, l'alcényle, l'alcinyle, l'aryle, l'hétérocycloalkyle ou l'hétéroaryle peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O(alkyle), O-(CH₂CH₂)ₙ-OH, O-(CH₂CH₂)ₙ-O(alkyle), O(aralkyle), O(aryle), OPO₃²⁻, PO₃²⁻ et SO₃⁻, ou
R⁹ et R¹⁰ forment, en commun avec l'atome N auquel ils sont liés, un hétérocycle pentagonal à heptagonal, dans lequel l'hétérocycle peut comprendre un autre hétéroatome sélectionné parmi le groupe constitué de N, O et S et/ou peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O(alkyle), O-(CH₂CH₂)ₙ-OH, O-(CH₂CH₂)ₙ-O(alkyle), O(aralkyle), O(aryle), OPO₃²⁻, PO₃²⁻ et SO₃⁻;
X représente un hétéroatome sélectionné parmi le groupe constitué de O et S ; et
n représente un nombre entier de 1 à 6 ;
sous réserve qu'au moins deux radicaux sélectionnés parmi le groupe constitué de R², R³, R⁴ et R⁵ représentent le radical H et le composé azo de formule générale (I) n'est pas du 2-(éthyl-{3-méthyl-4-[(pipéridine-1-ylcarbonyl)diazényl]phényl}amino)éthanol.

2. Composé azo selon la revendication 1,
**caractérisé en ce que**,
R¹ représente le radical NR⁷R⁸.

3. Composé azo selon la revendication 1 ou 2,
**caractérisé en ce que**,
au moins trois radicaux sélectionnés parmi le groupe constitué de R², R³, R⁴ et R⁵ représentent le radical H.

4. Composé azo selon l'une quelconque des revendications 1 à 3, **caractérisé en ce que**,
R² représente un radical sélectionné parmi le groupe constitué de H et O(alkyle), et R³, R⁴ et R⁵ représentent respectivement le radical H.

5. Composé azo selon l'une quelconque des revendications 1 à 4, **caractérisé en ce que**,
R⁶ représente le radical C(=X)NR⁹R¹⁰, en particulier le radical C(=O)NR⁹R¹⁰.

6. Composé azo selon l'une quelconque des revendications 1 à 5, **caractérisé en ce que**,
R⁸ comprend un ou plusieurs substituant(s) sélectionné(s) parmi le groupe constitué de COO⁻, N(alkyle)₃⁺, NH-C(=NH₂)-NH₂⁺, OH, O-(CH₂CH₂)ₙ-OH, O-(CH₂CH₂)ₙ-O(alkyle), OPO₃²⁻, PO₃²⁻ et SO₃⁻.

7. Composé azo selon l'une quelconque des revendications 1 à 6, **caractérisé en ce que**,
R⁷ et/ou R⁸ représente(nt) le radical hydroxyéthyle.

8. Composé azo selon l'une quelconque des revendications 1 à 7, **caractérisé en ce que**,
R⁹ et/ou R¹⁰ représente(nt) un radical sélectionné parmi le groupe constitué de alkyle et aryle, dans lequel l'alkyle ou l'aryle peut comprendre un ou plusieurs substituant(s) sélectionné(s) parmi le groupe constitué de OH, O(alkyle) et O(aryle), ou
R⁹ et R¹⁰ forment, en commun avec l'atome N auquel ils sont liés, un hétérocycle pentagonal ou hexagonal, dans lequel l'hétérocycle peut comprendre un autre hétéroatome sélectionné parmi le groupe constitué de N, O et S.

9. Composé azo selon l'une quelconque des revendications 1 à 8, **caractérisé en ce que**,
R⁹ et/ou R¹⁰ représente(nt) un radical sélectionné parmi le groupe constitué de méthyle, éthyle, phényle, hydroxyméthyle, hydroxyéthyle, hydroxyphényle et méthoxyphényle.

10. Utilisation d'un composé azo de formule générale (I) 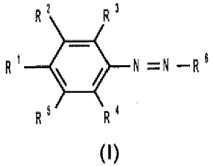 en tant que médiateur dans un procédé diagnostique, dans laquelle
R¹ représente un radical sélectionné parmi le groupe constitué de OH, OR⁷, SR⁷, NHR⁷, et NR⁷R⁸;
R², R³, R⁴ et R⁵ représentent, indépendamment les uns des autres, un radical sélectionné parmi le groupe constitué de H, alkyle, cycloalkyle, alcényle, alcinyle, aryle, hétérocycloalkyle, hétéroaryle, CN, COO⁻, halogène, O(alkyle), O(aryle), NH(alkyle), N(alkyle)₂, N(alkyle)₃⁺, NH(aryle), N(aryle)₂, NO₂, et SO₃⁻, dans laquelle l'alkyle, le cycloalkyle, l'alcényle, l'alcinyle, l'aryle, l'hétérocycloalkyle, l'hétéroaryle, l'O(alkyle), l'O(aryle), le NH(alkyle), le N(alkyle)₂, le N(alkyle)₃⁺, le NH(aryle) ou le N(aryle)₂ peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻;
R⁶ représente un radical sélectionné parmi le groupe constitué de CN, C(=X)NH₂, C(=X)NHR⁹, C(=X)NR⁹R¹⁰, C(=X)NHOH et C(=X)R⁹;
R⁷ et R⁸ représentent, indépendamment l'un de l'autre, un radical sélectionné parmi le groupe constitué de alkyle, cycloalkyle, alcényle, alcinyle, aryle, hétérocycloalkyle et hétéroaryle, dans laquelle l'alkyle, le cycloalkyle, l'alcényle, l'alcinyle, l'aryle, l'hétérocycloalkyle ou l'hétéroaryle peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻; ou
R⁷ et R⁸ forment, en commun avec l'atome N auquel ils sont liés, un hétérocycle pentagonal à heptagonal, dans laquelle l'hétérocycle peut comprendre un autre hétéroatome sélectionné parmi le groupe constitué de N, O et S et/ou peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻;
R⁹ et R¹⁰ représentent, indépendamment l'un de l'autre, un radical sélectionné parmi le groupe constitué de alkyle, cycloalkyle, alcényle, alcinyle, aryle, hétérocycloalkyle et hétéroaryle, dans laquelle l'alkyle, le cycloalkyle, l'alcényle, l'alcinyle, l'aryle, l'hétérocycloalkyle ou l'hétéroaryle peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻; ou
R⁹ et R¹⁰ forment, en commun avec l'atome N auquel ils sont liés, un hétérocycle pentagonal à heptagonal, dans laquelle l'hétérocycle peut comprendre un autre hétéroatome sélectionné parmi le groupe constitué de N, O et S et/ou peut comprendre un ou plusieurs substituant(s) sélectionné(s) parmi CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻;
X représente un hétéroatome sélectionné parmi le groupe constitué de O et S ; et
n représente un nombre entier de 1 à 6.

11. Réactif de détection destiné à déterminer la présence d'un analyte, comprenant
(a) une oxydoréductase flavine-dépendante, nicotinamide-dépendante ou pyrroloquinoléine quinone-dépendante,
(b) une coenzyme réductible de flavine, de nicotinamide, ou de pyrroloquinoléine quinone,
(c) un composé azo de formule générale (I) 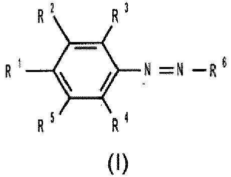 dans laquelle
R¹ représente un radical sélectionné parmi le groupe constitué de OH, OR⁷, SR⁷, NHR⁷et NR⁷R⁸;
R², R³, R⁴ et R⁵ représentent, indépendamment les uns des autres, un radical sélectionné parmi le groupe constitué de H, alkyle, cycloalkyle, alcényle, alcinyle, aryle, hétérocycloalkyle, hétéroaryle, CN, COO⁻, halogène, O(alkyle), O(aryle), NH(alkyle), N(alkyle)₂, N(alkyle)₃⁺, NH(aryle), N(aryle)₂, NO₂, et SO₃⁻, dans lequel l'alkyle, le cycloalkyle, l'alcényle, l'alcinyle, l'aryle, l'hétérocycloalkyle, l'hétéroaryle, l'O(alkyle), l'O(aryle), le NH(alkyle), le N(alkyle)₂, le N(alkyle)₃⁺, le NH(aryle) ou le N(aryle)₂ peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻;
R⁶ représente un radical sélectionné parmi le groupe constitué de CN, C(=X)NH₂, C(=X)NHR⁹, C(=X)NR⁹R¹⁰, C(=X)NHOH et C(=X)R⁹;
R⁷ et R⁸ représentent, indépendamment l'un de l'autre, un radical sélectionné parmi le groupe constitué de alkyle, cycloalkyle, alcényle, alcinyle, aryle, hétérocycloalkyle et hétéroaryle, dans lequel l'alkyle, le cycloalkyle, l'alcényle, l'alcinyle, l'aryle, l'hétérocycloalkyle ou l'hétéroaryle peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻, ou
R⁷ et R⁸ forment, en commun avec l'atome N auquel ils sont liés, un hétérocycle pentagonal à heptagonal, dans lequel l'hétérocycle peut comprendre un autre hétéroatome sélectionné parmi le groupe constitué de N, O et S et/ou peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻;
R⁹ et R¹⁰ représentent, indépendamment l'un de l'autre, un radical sélectionné parmi le groupe constitué de alkyle, cycloalkyle, alcényle, alcinyle, aryle, hétérocycloalkyle ou hétéroaryle, dans lequel l'alkyle, le cycloalkyle, l'alcényle, l'alcinyle, l'aryle, l'hétérocycloalkyle ou l'hétéroaryle peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, PO₃²⁻ et SO₃⁻, ou
R⁹ et R¹⁰ forment, en commun avec l'atome N auquel ils sont liés, un hétérocycle pentagonal à heptagonal, dans lequel l'hétérocycle peut comprendre un autre hétéroatome sélectionné parmi le groupe constitué de N, O et S et/ou peut comprendre un ou plusieurs substituant(s) sélectionné(s) parmi le groupe constitué de CN, COO⁻, halogène, NH₂, NH(alkyle), N(alkyle)₂, N(alkyle)₃⁺, NH(aryle), N(aryle)₂, NH-C(=NH₂)-NH₂⁺, NO₂, OH, O-(CH₂CH₂)ₙ-OH, O-(CH₂CH₂)ₙ-O(alkyle), O(alkyle), O(aralkyle), O(aryle), OPO₃²⁻, Po₃²⁻ et SO₃⁻ ;
X représente un hétéroatome sélectionné parmi le groupe constitué de O et S ; et
n représente un nombre entier de 1 à 6, et
(d) éventuellement un indicateur optique réductible.

12. Réactif de détection selon la revendication 11,
**caractérisé en ce que** l'oxydoréductase flavine-dépendante, nicotinamide-dépendante ou pyrroloquinoléine quinone-dépendante est une déshydrogénase flavine-dépendante, nicotinamide-dépendante ou pyrroloquinoléine quinone-dépendante, en particulier une glucose-déshydrogénase flavine-dépendante, nicotinamide-dépendante ou pyrroloquinoléine quinone-dépendante ou une glucose-6-phosphate-déshydrogénase,
et/ou
**en ce que** la coenzyme réductible de flavine, de nicotinamide ou de pyrroloquinoléine quinone correspond à FAD, FMN, NAD⁺, NADP⁺, PQQ ou un dérivé de ceux-ci, et correspond en particulier à NAD⁺, NADP⁺, carbaNAD⁺ ou carbaNADP⁺.

13. Trousse de détermination de la présence d'un analyte, comprenant
(a) un réactif de détection selon la revendication 11 ou 12, et
(b) un élément de test, en particulier un élément de test réalisé en tant que capteur optique ou électrochimique.

14. Élément de test pour la détermination d'un analyte, comprenant un réactif de détection selon la revendication 11 ou 12, dans lequel l'élément de test est réalisé de manière préférée en tant que capteur optique ou électrochimique.

15. Procédé de détermination d'un analyte, comprenant les étapes :
(a) mettre en contact un échantillon contenant l'analyte avec un réactif de détection selon la revendication 11 ou 12, une trousse selon la revendication 13, ou un élément de test selon la revendication 14, et
(b) déterminer la présence et/ou la quantité de l'analyte.
